# EUROPEAN PATENT SPECIFICATION

(11) **EP 4 392 121 B1**
(45) Date of publication and mention of the grant of the patent: **08.04.2026**
(21) Application number: 22862161.1
(22) Date of filing: 26.08.2022
(51) Int. Cl.: A61N 1/04, A61N 1/32, A61N 1/36, A61B 5/01, A61B 5/0531, A61B 5/00

(54) **WIRELESS CLOSED-LOOP SMART BANDAGE FOR CHRONIC WOUND MANAGEMENT AND ACCELERATED TISSUE REGENERATION**
DRAHTLOSE INTELLIGENTE BANDAGE MIT GESCHLOSSENEM REGELKREIS FÜR CHRONISCHE WUNDBEHANDLUNG UND BESCHLEUNIGTE GEWEBEREGENERATION
BANDAGE INTELLIGENT SANS FIL À BOUCLE FERMÉE POUR LA GESTION DE PLAIES CHRONIQUES ET LA RÉGÉNÉRATION TISSULAIRE ACCÉLÉRÉE

(30) Priority: 27.08.2021 US 202163238017 P
(43) Date of publication of application: 03.07.2024
(73) Proprietor: The Board of Trustees of the Leland Stanford Junior University, Stanford, CA 94305-2038 (US)
(72) Inventor: JIANG, Yuanwen, Stanford, California 94305 (US); TROTSYUK, Artem, Stanford, California 94305 (US); BAO, Zhenan, Stanford, California 94305 (US); GURTNER, Geoffrey, Stanford, California 94305 (US); NIU, Simiao, Stanford, California 94305 (US)
(74) Representative: Forresters IP LLP
(86) International application number: PCT/US2022/041756
(87) International publication number: WO 2023/028349

(56) References cited:
- WO-A1-2014/078815
- US-A1- 2015 182 746
- US-A1- 2017 020 391
- US-A1- 2018 055 359
- US-A1- 2020 061 379
- US-B2- 10 058 455
- ISABEL DEL ET AL: "Conducting polymer materials for bioelectronics applications", 2 February 2018 (2018-02-02), XP055704165, Retrieved from the Internet <URL:https://addi.ehu.es/handle/10810/34588> [retrieved on 20200611]
- XU GANG, LU YANLI, CHENG CHEN, LI XIN, XU JIE, LIU ZHAOYANG, LIU JINGLONG, LIU GUANG, SHI ZHENGHAN, CHEN ZETAO, ZHANG FENNI, JIA Y: "Battery‐Free and Wireless Smart Wound Dressing for Wound Infection Monitoring and Electrically Controlled On‐Demand Drug Delivery", ADVANCED FUNCTIONAL MATERIALS, WILEY - V C H VERLAG GMBH & CO. KGAA, DE, vol. 31, no. 26, 1 June 2021 (2021-06-01), DE , pages 2100852, XP093038718, ISSN: 1616-301X, DOI: 10.1002/adfm.202100852
- ZHANG JIEYU, WU CAN, XU YUANYUAN, CHEN JIALI, NING NING, YANG ZEYU, GUO YI, HU XUEFENG, WANG YUNBING: "Highly Stretchable and Conductive Self-Healing Hydrogels for Temperature and Strain Sensing and Chronic Wound Treatment", APPLIED MATERIALS & INTERFACES, AMERICAN CHEMICAL SOCIETY, US, vol. 12, no. 37, 16 September 2020 (2020-09-16), US , pages 40990 - 40999, XP093038716, ISSN: 1944-8244, DOI: 10.1021/acsami.0c08291

## Description

### TECHNICAL FIELD

The present embodiments relate generally to health treatment, and more particularly to a skin-conformal wireless smart bandage having a built-in closed-loop feedback to actively deliver precise electrical stimulation in response to the detection of early infection after hemostasis has been achieved.

### BACKGROUND

Wound infections are a leading cause of chronic non-healing wounds. It is estimated that wound infections occur in 15-25% of all wounds and result in morbidity (i.e., sepsis) and even mortality in vulnerable populations, such as diabetics. There remains a pressing need to develop therapeutic devices to rapidly detect and treat local wound infection before they become clinically obvious. Wearable devices are well positioned to address these challenges by integrating biosensors to detect wound infection and then deliver active wound care treatment without requiring the intervention of a physician. US patent application publication US2020061379A1 discloses an apparatus comprising a smart bandage that includes skin electrodes comprising a conducting hydrogel configured to provide skin adhesion for the smart bandage; and a flexible electronics having processing circuitry and a wireless interface for communicating with external processors. A need remains, however, for a more ideal wound care technology that would be able to (i) detect local wound infection at its earliest stages and (ii) automatically initiate treatment in a closed-loop fashion.

It is against this technological backdrop that the present Applicant sought to address these and other technological issues deeply rooted in this technology.

### SUMMARY

The present invention is defined by the appended claims. Aspects, embodiments and examples described hereinafter are only of exemplary nature and are presented for better understanding the present invention. The present embodiments relate to a skin-conformal wireless smart bandage that includes a built-in closed-loop feedback to actively deliver precise electrical stimulation in response to the detection of early infection after hemostasis has been achieved. The intermittent and episodic nature of this closed-loop operation allows the device to function for at least 5-7 days, consistent with current clinical practice in wound care. This is done in some embodiments by using a combination of (1) conductive hydrogels, (2) flexible electronics and (3) on-board processing circuitry.

One or more embodiments use reversible skin-adhesive electrodes which possess good skin adhesion, robustness, and low impedance for electrical stimulation. These and other embodiments use an electronic-ionic dual conducting polymer complex consisting of polymerized biocompatible (meth)acrylate monomers in the presence of conducting polymers based on PEDOT:PSS with ultralow stiffness (modulus 1~10 kPa) and high conductivity (up to 50 S/cm). Compared to traditional ionically conducting hydrogels, this polymer-based dual-conducting hydrogel has lower impedance across the entire frequency domain translating to more efficient sensing and stimulation. For high tissue adhesion, these and other embodiments incorporate adhesive interfacial chemistry (hydrogen bonding, metal-coordination), and tuned nanoscopic intermolecular interaction (crosslinking density). To avoid secondary skin damage due to adhesive electrodes, reversible hydrogel chemistry was developed to modulate gel adhesion (temperature triggered phase change, chemical triggered debonding).

One example wireless smart bandage consists of a flexible circuit containing existing biosensor technology for impedance and temperature with electrical stimulation capable to be controlled by a processing unit. The fully integrated flexible circuit of the smart bandage consists of an antenna for wireless energy harvesting or a rechargeable battery for energy supply, ring oscillator for AC impedance measurement, temperature sensitive resistor for temperature sensing, a diode rectifier for potentiometric electrochemical sensing, a microcontroller for data processing, and a Bluetooth unit for wireless communication.

In embodiments, the hydrogel is integrated to the processing core through gold wires. These wires are safer than magnesium-based wires and will not risk local toxicity and subsequent tissue necrosis. Once the hydrogel is connected to the processing core, the smart bandage is then outfitted with wireless sensors. These units allow for mobility and enable patients to resume normal daily activity. They consist of a disposable battery with cobalt cathodes for the power supply and a microcontroller for data collection and analysis. The central processing core is encapsulated in a protective casing, which is attachable to the flexible bandage and gel electrodes.

The initial sensing and stimulation thresholds can be set by utilizing sensing data gathered from commercial sensing instruments (impedance, temperature) on human wounds and healed skin through an approved IRB. Embodiments then use a binary decision classifier to create a decision tree through which the microcontroller can operate. Episodic sensing occurs over 5 second intervals. If an abnormal spike in temperature or impedance occurs, the stimulation circuits will activate electrical pulses. Pulse duration and frequency can be dependent on the severity of the impendence/temperature spike (e.g. a more healed wound can get less treatment than a non-healed one). The battery-powered processing core can be rechargeable, allowing for the attachment of a disposable wound dressing along with a reusable core. By utilizing a simple algorithm, power consumption can be minimized, thereby maximizing sensing and stimulation output for extended wear. To optimize data transfer, information can be relayed back via Bluetooth to a local computer.

A smart bandage according to some embodiments resembles a wound dressing and can be integrated such that the sensors are in contact with the fluid absorbed in the dressing while the electrical stimulation skin-adhesive electrodes are placed on either side of the wound. The soft and elastic hydrogel-based microelectrodes will form a stable interface with the skin, enabling an efficient signal exchange and energy delivery. The results in a biocompatible soft bioelectronic system that can be applied to not only wound infection but to potentially other adjacent fields where a sensor-actuator closed loop system might be useful.

### BRIEF DESCRIPTION OF THE DRAWINGS

These and other aspects and features of the present embodiments will become apparent to those ordinarily skilled in the art upon review of the following description of specific embodiments in conjunction with the accompanying figures, wherein:
Figures 1a to 1f illustrate aspects of an example design of a wireless smart bandage for chronic wound management according to embodiments.
Figures 2a to 2i illustrate aspects of example wireless sensing and stimulation circuits according to embodiments.
Figures 3a to 3m illustrate aspects of an example tough and low-impedance conductive hydrogel electrode with reversible tissue adhesion according to embodiments.
Figures 4a to 4l illustrate aspects of how a wireless smart bandage system according to embodiments can continuously monitor physiological wound conditions and accelerate tissue regeneration.
Figures 5a to 5k illustrate aspects of a molecular mechanism attributing to the accelerated tissue regeneration with electrical stimulation in example embodiments.
Figure 6 is a graph illustrating an example loading curve of the antenna wireless energy harvesting.
Figure 7a to 7g illustrates aspects of an example high-pass filter (HPF) design for impedance sensing according to embodiments.
Figures 8a and 8b illustrate antenna characterization under bending according to embodiments.
Figures 9a to 9c illustrate aspects of example calibration of the sensors and wireless readout of temperature upon heating.
Figures 10a to 10d illustrate an example device architecture of the wireless smart bandage according to embodiments.
Figure 11 illustrates closed-loop operation of an example wireless smart bandage according to embodiments.
Figures 12a to 12d illustrate an example comparison of the gelation outcomes using different initiators.
Figures 13a are 13b are example EIS measurements and electrical conductivity measurements, respectively, of the hydrogel electrode with different concentrations of PEDOT:PSS according to embodiments.
Figures 14a and 14b are example Chronoamperometry measurements and injected current measurements, respectively, of the hydrogel electrode with different concentrations of PEDOT:PSS according to embodiments.
Figures 15a and 15b are cyclic tests of EIS and chronoamperometry, respectively, of the hydrogel electrode according to embodiments.
Figures 16a and 16b illustrate Uni-directional tensile tests of the composite hydrogel electrode according to embodiments.
Figures 17a and 17b illustrate cyclic loading of the hydrogel electrode and corresponding impedance changes according to embodiments.
Figures 18a and 18b illustrate an example 180-degree peeling test of the hydrogel adhesion onto various substrates according to embodiments.
Figures 19a to 19c illustrate aspects of Stable hydrogel adhesion and impedance sensing with simulated sweat according to embodiments.
Figure 20 provides Photos showing the tunable adhesion of the same hydrogel electrode with a hot plate under different temperature according to embodiments.
Figure 21 provides Photos showing the tunable adhesion of the same hydrogel electrode with mouse skin under different temperature according to embodiments.
Figure 22 provides Photos showing minimal changes of the hydrogel adhesion at different temperatures below the LCST phase transition point according to embodiments.
Figure 23a to 23d illustrate aspects of Minimal mutual interference of the sensor functions according to embodiments.
Figure 24 illustrates Semi-quantitative scoring criteria for paraffin sections to assess the hydrogel biocompatibility.
Figures 25a to 25c illustrate aspects of an example Hydrogel electrode showing excellent biocompatibility after long-term contact with mouse skin according to embodiments.
Figures 26a and 26b illustrate example Impedance and temperature signals, respectively, recorded over time for mice with and without treatment according to embodiments.
Figures 27a and 27b illustrate aspects of how Electrical stimulation induces higher skin toughness after healing compared to the control tissue according to embodiments.
Figures 28a to 28c illustrate aspects of how a Wireless smart bandage according to embodiments allows longer treatment per day with better healing outcomes.
Figures 29a and 29b illustrate example Histological studies showing improved wound healing outcomes for excisional wounds treated with electrical stimulation according to embodiments.
Figures 30a and 30b illustrate aspects of a FracLac evaluation showing less scar-like fiber arrangement for excisional wounds treated with electrical stimulation after 13 days according to embodiments.
Figures 31a and 31b illustrates aspects of Picrosirius red staining showing more random collagen networks for excisional wounds treated with electrical stimulation after 13 days according to embodiments.
Figures 32a to 32c are Histology and immunostaining images revealing more neovascularization for excisional wounds treated with electrical stimulation after 13 days according to embodiments.
Figures 33a to 33c illustrate aspects of Histological studies showing improved healing outcomes for burn wounds treated with electrical stimulation after 19 days according to embodiments.
Figures 34a and 34b illustrate aspects of a FracLac evaluation showing less scar-like fiber arrangement for burn wounds treated with electrical stimulation after 19 days according to embodiments.
Figures 35a and 35b illustrate aspects of Picrosirius red staining showing more random collagen networks for burn wounds treated with electrical stimulation after 19 days according to embodiments.
Figures 36a to 36c illustrate aspects of Histology and immunostaining studies revealing more neovascularization for burn wounds treated with electrical stimulation after 19 days according to embodiments.
Figures 37a to 37e illustrate aspects of Closed-loop detection and treatment of early infection in a wound according to embodiments.
Figures 38a to 38d illustrate aspects of Histological studies showing improved healing outcomes for STZ-induced diabetic excisional wound model treated with electrical stimulation after 15 days according to embodiments.
Figures 39a and 39b illustrate aspects of a FracLac evaluation showing less scar-like fiber arrangement for STZ-induced diabetic wounds treated with electrical stimulation after 15 days according to embodiments.
Figures 40a and 40b illustrate aspects of Picrosirius red staining showing more random collagen networks for STZ-induced diabetic excisional wounds treated with electrical stimulation after 15 days according to embodiments.
Figures 41a to 41c illustrate aspects of Histology and immunostaining studies revealing more neovascularization for STZ-induced diabetic excisional wounds treated with electrical stimulation after 15 days according to embodiments.
Figure 42 provides Fluorescent imaging showing the alignment of human umbilical vein endothelial cells (HUVEC) under electric field (1 V cm⁻¹) according to embodiments.
Figure 43 provides graphs illustrating Trajectories of individual HUVEC cells showing directional migration under electric field (1 V cm⁻¹) according to embodiments.
Figures 44a and 44b provide a Quantitative comparison of the total number of cells from tissue with and without stimulation after 5 days and relative percentages for each cell type, respectively, according to embodiments.
Figure 45 provides Volcano plots showing the number of genes with significant differences for each cell type according to embodiments presented in Fig. 5c.
Figures 46a and 46b are plots showing the trajectory of RNA velocity according to embodiments shown in Fig. 5e.
Figure 47 illustrates Relative percentage showing the ratio of cell counts in the stimulated and control groups for each Seurat cluster in the macrophage and monocyte group presented in Fig. 5f.
Figure 48 provides Violin plots showing the relative expression profiles of pro-regenerative genes in the stimulated and control groups within the macrophage and monocyte Seurat cluster presented in Fig. 5h.
Figure 49 are graphs illustrating a Gating strategy for the fluorescence-activated cell sorting (FACS) of cells in the excisional wound of the wild-type mouse in the GFP+/WT parabiosis model as shown in Fig. 5i.

### DETAILED DESCRIPTION

The present embodiments will now be described in detail with reference to the drawings, which are provided as illustrative examples of the embodiments so as to enable those skilled in the art to practice the embodiments and alternatives apparent to those skilled in the art. Moreover, where certain elements of the present embodiments can be partially or fully implemented using known components, only those portions of such known components that are necessary for an understanding of the present embodiments will be described, and detailed descriptions of other portions of such known components will be omitted so as not to obscure the present embodiments. Embodiments described as being implemented in software should not be limited thereto, but can include embodiments implemented in hardware, or combinations of software and hardware, and vice-versa, as will be apparent to those skilled in the art, unless otherwise specified herein. In the present specification, an embodiment showing a singular component should not be considered limiting; rather, the present disclosure is intended to encompass other embodiments including a plurality of the same component, and vice-versa, unless explicitly stated otherwise herein. Moreover, applicants do not intend for any term in the specification or claims to be ascribed an uncommon or special meaning unless explicitly set forth as such. Further, the present embodiments encompass present and future known equivalents to the known components referred to herein by way of illustration.

### Introduction

Chronic non-healing wounds represent a significant healthcare burden, with more than 6 million individuals affected in the United States alone (Han, G. & Ceilley, R. Chronic Wound Healing: A Review of Current Management and Treatments. Adv Ther 34, 599-610, doi:10.1007/s12325-017-0478-y (2017)). A chronic wound is defined as a wound that has failed to heal by 8-12 weeks and is unable to restore function and anatomical integrity to the affected site (Werdin, F., Tenenhaus, M. & Rennekampff, H.-O. Chronic wound care. The Lancet 372, 1860-1862, doi:10.1016/s0140-6736(08)61793-6 (2008)). These wounds are associated with loss of function and mobility, increased social stress and isolation, depression and anxiety, prolonged hospitalization, and overall increased morbidity and mortality. In addition, the financial cost to the healthcare system for the management of chronic wound-related complications has been estimated to exceed $25 billion annually.

In normal wound healing, when an injury occurs, the tissue undergoes three canonical stages of wound regeneration: inflammation, new tissue formation, and remodeling (Gurtner, G. C., Werner, S., Barrandon, Y. & Longaker, M. T. Wound repair and regeneration. Nature 453, 314-321, doi:10.1038/nature07039 (2008)). During each stage, different cells are recruited, migrate, become activated, and proliferate to achieve tissue regeneration and reduce infection (Martin, P. Wound healing--aiming for perfect skin regeneration. Science 276, 75-81, doi:10.1126/science.276.5309.75 (1997)). When this carefully orchestrated process is impaired, there is often not typically a single cause, but rather multiple factors, that contribute. These factors include comorbidities such as diabetes, infection, ischemia, metabolic conditions, immunosuppression, and radiation, which can result in high level of proteases, elevated inflammatory markers, low growth factor activity, and reduced cellular proliferation within the wound bed. This can lead to significant patient discomfort and increased hospitalization rates (Singer, A. J. & Clark, R. A. Cutaneous wound healing. N Engl J Med 341, 738-746, doi:10.1056/NEJM199909023411006 (1999); Frykberg, R. G. & Banks, J. Challenges in the Treatment of Chronic Wounds. Adv Wound Care (New Rochelle) 4, 560-582, doi:10.1089/wound.2015.0635 (2015)).

While interventions for chronic wounds exist, such as growth factors, extracellular matrix, engineered skin, and negative pressure wound therapy, these treatments are only moderately effective (Frykberg, R. G. & Banks, J. Challenges in the Treatment of Chronic Wounds. Adv Wound Care (New Rochelle) 4, 560-582, doi:10.1089/wound.2015.0635 (2015); Rodrigues, M., Kosaric, N., Bonham, C. A. & Gurtner, G. C. Wound Healing: A Cellular Perspective. Physiol Rev 99, 665-706, doi:10.1152/physrev.00067.2017 (2019)). Current standard-of-care wound dressings are passive and do not actively respond to variations in the wound environment. Smart bandage technologies are well positioned to address these challenges with their ability to integrate multimodal sensors and stimulators for real-time monitoring and active wound care treatment with minimal physician intervention (McLister, A., McHugh, J., Cundell, J. & Davis, J. New Developments in Smart Bandage Technologies for Wound Diagnostics. Adv Mater 28, 5732-5737, doi:10.1002/adma.201504829 (2016); Derakhshandeh, H., Kashaf, S. S., Aghabaglou, F., Ghanavati, I. O. & Tamayol, A. Smart Bandages: The Future of Wound Care. Trends Biotechnol 36, 1259-1274, doi:10.1016/j.tibtech.2018.07.007 (2018); Long, Y. et al. Effective Wound Healing Enabled by Discrete Alternative Electric Fields from Wearable Nanogenerators. ACS Nano 12, 12533-12540, doi:10.1021/acsnano.8b07038 (2018); Liu, A. et al. Accelerated complete human skin architecture restoration after wounding by nanogenerator-driven electrostimulation. J Nanobiotechnology 19, 280, doi:10.1186/s12951-021-01036-7 (2021); Farahani, M. & Shafiee, A. Wound Healing: From Passive to Smart Dressings. Adv Healthc Mater 10, e2100477, doi:10.1002/adhm.202100477 (2021); Dincer, C. et al. Disposable Sensors in Diagnostics, Food, and Environmental Monitoring. Adv Mater 31, e1806739, doi:10.1002/adma.201806739 (2019); Barros Almeida, I. et al. Smart Dressings for Wound Healing: A Review. Adv Skin Wound Care 34, 1-8, doi:10.1097/01.ASW.0000725188.95109.68 (2021)).

Among other aspects, the present Applicant recognizes that as a wound heals, skin impedance increases. When a wound becomes infected, however, wound impedance would decrease due to the development of biofilm (Lukaski, H. C. & Moore, M. Bioelectrical impedance assessment of wound healing. J Diabetes Sci Technol 6, 209-212, doi:10.1177/193229681200600126 (2012)). As the infection develops further, local inflammation increases wound temperature (Chanmugam, A. et al. Relative Temperature Maximum in Wound Infection and Inflammation as Compared with a Control Subject Using Long-Wave Infrared Thermography. Adv Skin Wound Care 30, 406-414, doi:10.1097/01.ASW.0000522161.13573.62 (2017)). The present Applicant further recognizes that both signals can be easily captured by low-cost sensors embedded in a wearable device to act as a sentinel for impending wound infection. These biophysical signals provide rapid, robust, and accurate information about wound conditions in real time, creating an opportunity to diagnose and monitor a non-healing wound quickly and autonomously in a closed-loop fashion.

Current smart bandage technologies have demonstrated promise in their ability to sense physiological conditions. This includes detecting pH (Tamayol, A. et al. Flexible pH-Sensing Hydrogel Fibers for Epidermal Applications. Adv Healthc Mater 5, 711-719, doi:10.1002/adhm.201500553 (2016); Xu, G. et al. Battery-Free and Wireless Smart Wound Dressing for Wound Infection Monitoring and Electrically Controlled On-Demand Drug Delivery. Advanced Functional Materials 31, 2100852, doi:10.1002/adfm.202100852 (2021)), temperature (Trung, T. Q., Ramasundaram, S., Hwang, B. U. & Lee, N. E. An All-Elastomeric Transparent and Stretchable Temperature Sensor for Body-Attachable Wearable Electronics. Adv Mater 28, 502-509, doi:10.1002/adma.201504441 (2016); Hattori, Y. et al. Multifunctional skin-like electronics for quantitative, clinical monitoring of cutaneous wound healing. Adv Healthc Mater 3, 1597-1607, doi:10.1002/adhm.201400073 (2014); Shi, X. & Wu, P. A Smart Patch with On-Demand Detachable Adhesion for Bioelectronics. Small 17, e2101220, doi:10.1002/smll.202101220 (2021); Pang, Q. et al. Smart Flexible Electronics-Integrated Wound Dressing for Real-Time Monitoring and On-Demand Treatment of Infected Wounds. Adv Sci (Weinh) 7, 1902673, doi:10.1002/advs.201902673 (2020)), oxygenation (Marks, H. et al. A paintable phosphorescent bandage for postoperative tissue oxygen assessment in DIEP flap reconstruction. Sci Adv 6, doi:10.1126/sciadv.abd1061 (2020)), impedance (Swisher, S. L. et al. Impedance sensing device enables early detection of pressure ulcers in vivo. Nat Commun 6, 6575, doi:10.1038/ncomms7575 (2015); Mc Caffrey, C., Flak, J., Kiri, K. & Pursula, P. Flexible bioimpedance spectroscopy system for wound care monitoring. 1-4, doi:10.1109/biocas.2019.8919095 (2019)), motions (Kalidasan, V. et al. Wirelessly operated bioelectronic sutures for the monitoring of deep surgical wounds. Nat Biomed Eng 5, 1217-1227, doi:10.1038/s41551-021-00802-0 (2021); Zhao, Y. et al. Skin-Inspired Antibacterial Conductive Hydrogels for Epidermal Sensors and Diabetic Foot Wound Dressings. Advanced Functional Materials 29, 1901474, doi:10.1002/adfm.201901474 (2019)), and enzymatic fluctuations (Ciani, I. et al. Development of immunosensors for direct detection of three wound infection biomarkers at point of care using electrochemical impedance spectroscopy. Biosens Bioelectron 31, 413-418, doi:10.1016/j.bios.2011.11.004 (2012); Gao, Y. et al. A flexible multiplexed immunosensor for point-of-care in situ wound monitoring. Sci Adv 7, doi:10.1126/sciadv.abg9614 (2021)) of the wound. It has also been well established that electrical stimulation can reduce bacterial colonization, biofilm infection and restore normal wound healing in vivo (Thakral, G. et al. Electrical stimulation to accelerate wound healing. Diabet Foot Ankle 4, doi:10.3402/dfa.v4i0.22081 (2013)). Moreover, electrical stimulation has also been shown to improve tissue perfusion, stimulate immune cell function, and accelerate keratinocyte migration through a process known as galvanotaxis (Kloth, L. C. Electrical Stimulation Technologies for Wound Healing. Adv Wound Care (New Rochelle) 3, 81-90, doi:10.1089/wound.2013.0459 (2014); Zhao, M. et al. Electrical signals control wound healing through phosphatidylinositol-3-OH kinase-gamma and PTEN. Nature 442, 457-460, doi:10.1038/nature04925 (2006); Cohen, D. J., Nelson, W. J. & Maharbiz, M. M. Galvanotactic control of collective cell migration in epithelial monolayers. Nat Mater 13, 409-417, doi:10.1038/nmat3891 (2014)). Unfortunately, current electrical stimulation devices are bulky, tethered by wires, and uncomfortable to wear, limiting patient compliance. Although recent attempts have tried to generate electric fields by mechanical motions and subsequently accelerate wound healing using nanogenerator devices (see above), there have not been significant advancements in incorporating both sensing and electrical stimulation technologies to simultaneously deliver active wound care. Additionally, random mechanical movements cannot guarantee controllable stimulation conditions for consistent treatment. Further, metal-based electrodes without proper tissue adhesion could not ensure robust device operation in the complex wound environment. As a result, there remains a need to develop portable, autonomous, inexpensive devices to improve wound care.

Among other things, the present Applicant recognizes that for improved therapeutic outcomes, an ideal smart bandage platform needs to meet the following requirements. First, it needs to be flexible and wirelessly operated to avoid any undesired tethering and discomfort caused by conventional rigid, battery powered devices. Next, it should integrate both sensing and stimulation modalities for autonomous, closed-loop wound management. Finally, it should have on-demand skin adhesion with a tight interface for robust signal transduction and energy delivery during operation, while providing easy detachment to avoid possible secondary skin damage during device removal.

To address these requirements, the present Applicant developed a battery-free flexible bioelectronic system consisting of wirelessly powered sensing and stimulation circuits with tissue-interfacing tough hydrogel electrodes using a biocompatible conducting polymer. It is anticipated that this smart bandage will improve therapeutic outcomes and provide new knowledge for wound care.

Specifically, some embodiments include a miniaturized flexible printed circuit board (FPCB) containing an energy harvesting antenna, a microcontroller unit, a crystal oscillator, and filter circuits for dual channel continuous sensing of wound impedance and temperature, as well as a parallel stimulation circuit to deliver programmed electrical cues for accelerated wound healing. To ensure efficient signal exchange and energy delivery between the circuits and the soft skin tissue, a low-impedance and adhesive hydrogel electrode based on poly(3,4-ethylenedioxythiophene):polystyrene sulfonate (PEDOT:PSS) is provided. Compared to well-established ionically conducting hydrogels, the present dual-conducting (i.e. both electrically and ionically conductive) hydrogel has lower impedance across the entire frequency domain, giving rise to more efficient charge injection during stimulation (Liu, Y. et al. Soft and elastic hydrogel-based microelectronics for localized low-voltage neuromodulation. Nat Biomed Eng 3, 58-68, doi:10.1038/s41551-018-0335-6 (2019); Jiang, Y. et al. Topological supramolecular network enabled high-conductivity, stretchable organic bioelectronics. Science 375, 1411-1417, doi:10.1126/science.abj7564 (2022)). To mitigate secondary skin damage when peeling off the adhesive electrodes, some embodiments add a thermally controlled reversible phase transition mechanism to the hydrogel backbone that achieves two orders of magnitude lower adhesion at elevated temperature when compared to the normal skin temperature. Using multiple pre-clinical animal models, it was found that the present smart bandage could continuously monitor skin physiological signals and deliver directional electrical cues, leading to accelerated wound closure, increased neovascularization, and enhanced dermal recovery. Finally, the wireless nature of our smart bandage allowed utilizing complex animal models, such as parabiosis, to investigate the possible underlying mechanisms behind the observed effect of electrical stimulation. Data suggests that the beneficial wound healing outcomes could be attributed to the activation of pro-regenerative genes in the monocyte and macrophage cell populations.

### System overview

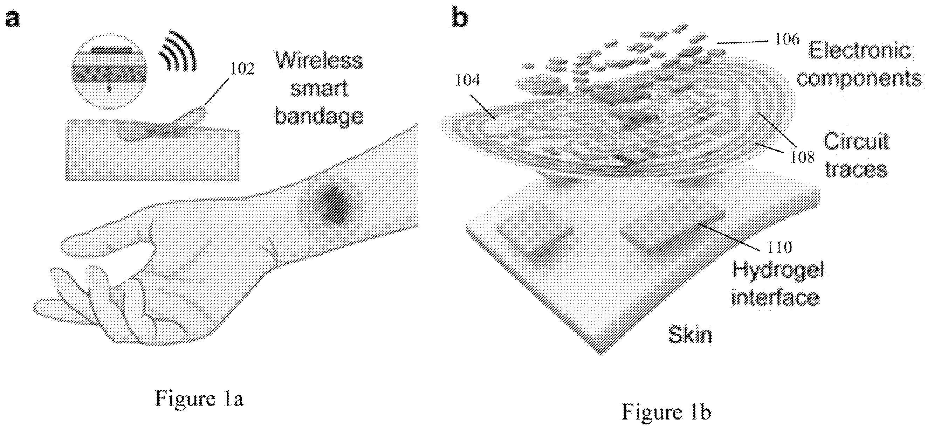

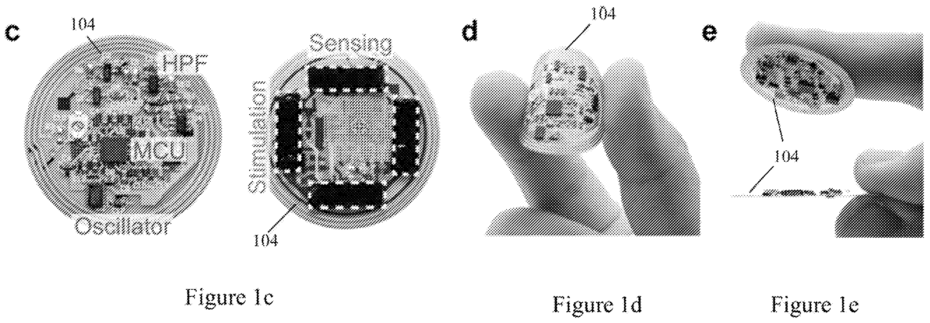

An example integrated wound management system according to embodiments is shown in Figures 1a to 1f. As shown, it includes a battery-free, wirelessly powered FPCB for simultaneous wound treatment and monitoring, as well as a tissue-interfacing conducting adhesive hydrogel interface for robust and gentle skin integration. Figure 1a is a diagram illustrating an example wireless smart bandage 102 according to embodiments and Figure 1b is an exploded view of the wireless smart bandage including flexible printed circuit board (FPCB) 104 comprising electronic components 106 and circuit traces 108, and a tissue-interfacing conducting adhesive hydrogel 106 according to embodiments. Figure 1c provides photographs of the front (left) and back (right) sides of the FPCB 104 showing a microcontroller unit (MCU), crystal oscillator, high-pass filter (HPF), and stimulation and sensing electrodes. Figure 1d provides photographs showing the flexibility of the FPCB, and Figure 1e illustrates the adhesion of the hydrogel interface (upper), and the thin layout of the board (lower). Due to the thin layout of the FPCB (~100 µm board thickness) and low modulus of the gel interface, the smart bandage is flexible and can be conformably attached to wound surfaces.

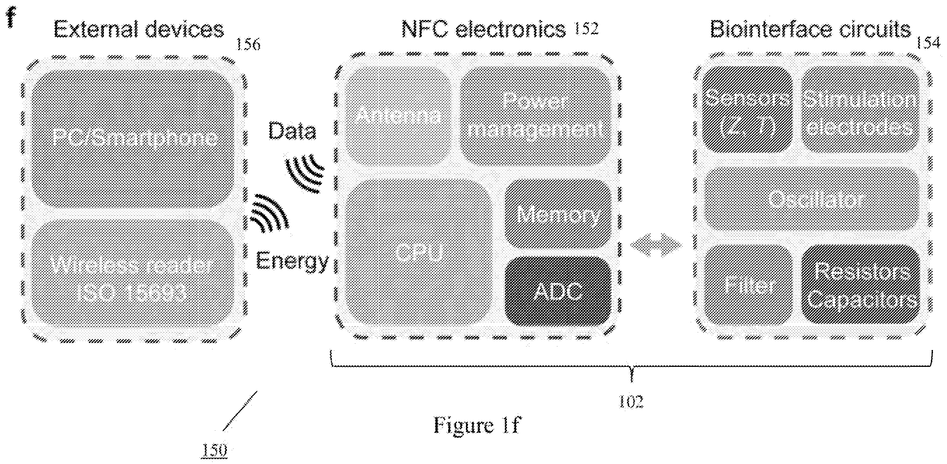

Figure 1f is a functional block diagram illustrating the components of an example wireless smart bandage system 150 according to embodiments, composed of near-field communication (NFC) electronics 152 with an interface to parallel stimulation and sensing modalities in biointerface circuits 154, as well as to a wireless interface to external devices 156 (e.g. external to the wireless smart bandage 102 itself). In this example, NFC electronics 152 includes a central processing unit (CPU) and analog-digital converter (ADC). With an antenna coil that resonates at 13.56 MHz, the electronics 152 can be inductively coupled with an external radiofrequency identification (RFID) reader in external devices 156. Through the RF energy harvesting process, the antenna can provide power to apply electric bias across the wound for programmed treatment and, at the same time, drive the microcontroller unit (MCU) and other integrated circuits (e.g., oscillator and filter), for continuous monitoring of wound impedance and temperature via a near-field communication (NFC) transponder in the MCU under the ISO15693 protocol.

### Wireless circuit design

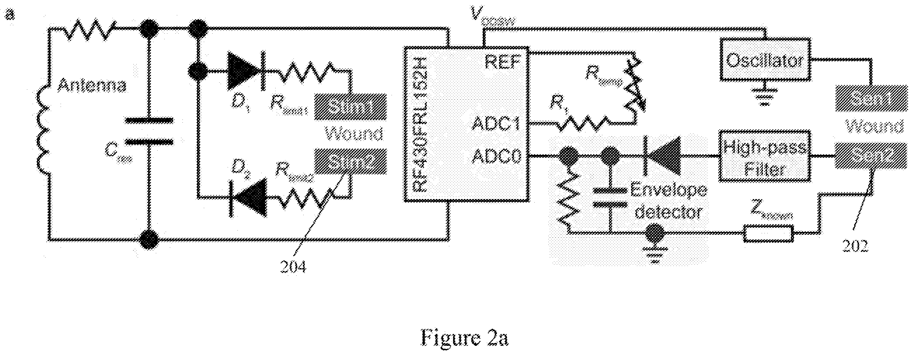

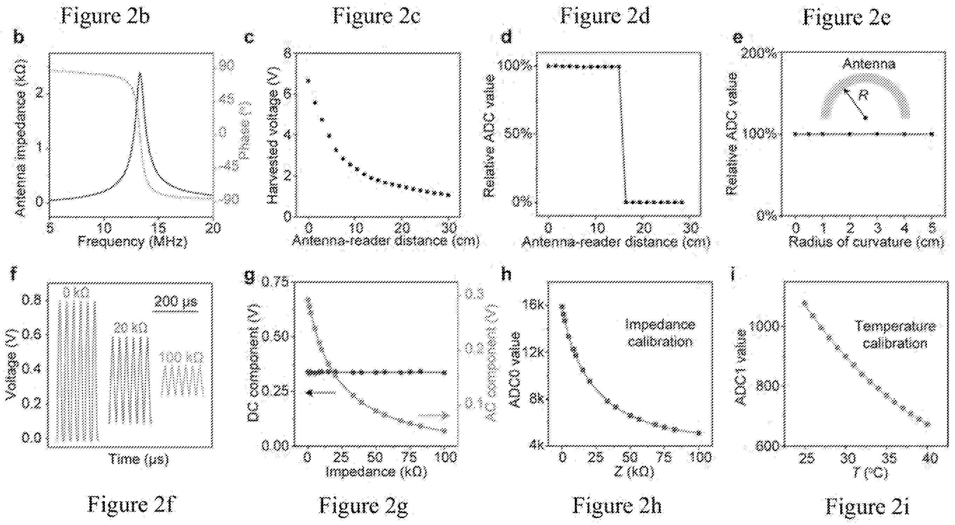

Figure 2a is an example circuit diagram of the wireless smart bandage 102 having components for simultaneous sensing 202 and stimulation 204. Figure 2b is a graph illustrating antenna resonant frequency and quality factor, and Figure 2c is a graph illustrating measured RF harvested voltage as a function of antenna-reader distance, both as measured from an example wireless smart bandage by a vector network analyzer (VNA).. Figure 2d is a graph illustrating that an example wireless readout operation from the microcontroller can function stably up to 15 cm away from the external reader in some embodiments. Figure 2e is a graph illustrating that wireless sensing can remain stable with bending radius down to 0.5 cm in some embodiments. Figure 2f is a graph illustrating voltage output after the high-pass filter showing reduced AC amplitudes with respect to larger resistance values in some example embodiments. Figure 2g is a graph that illustrates that in the meantime of Figure 2f, the DC component of the signals remain constant for all resistors tested. Figures 2h and 2i are graphs providing calibration curves of ADC values under known impedance (h) and temperature (i) in some embodiments.

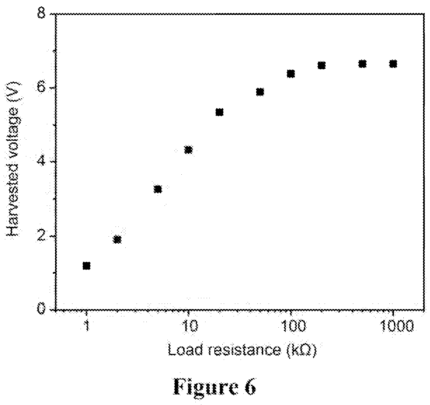

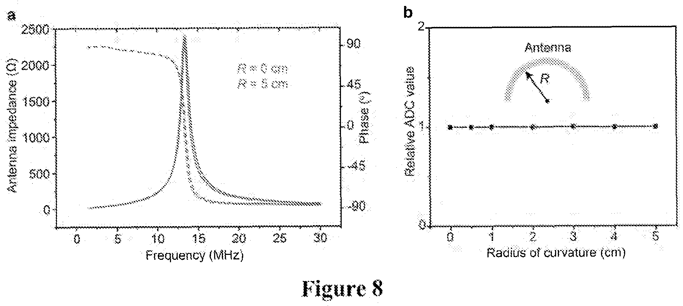

For the wireless antenna, some embodiments include a 5-turn coil with an optimum inductance of ~1.5 µH, offering a high RF harvested voltage and wide tunability to reach a resonant frequency of 13.56 MHz for maximized wireless communication signal gain as illustrated in Figure 6. Additionally, the quality factor (Q) of the antenna in these and other embodiments is ~18, which strikes the balance between energy harvesting efficiency and wireless communication bandwidth as illustrated in Figure 2b. As a result, the antenna offers a wide and stable 15 cm wireless communication distance as illustrated in Figures 2c and 2d. The device function of embodiments also remains stable upon bending as illustrated in Figures 2e and Figure 8.

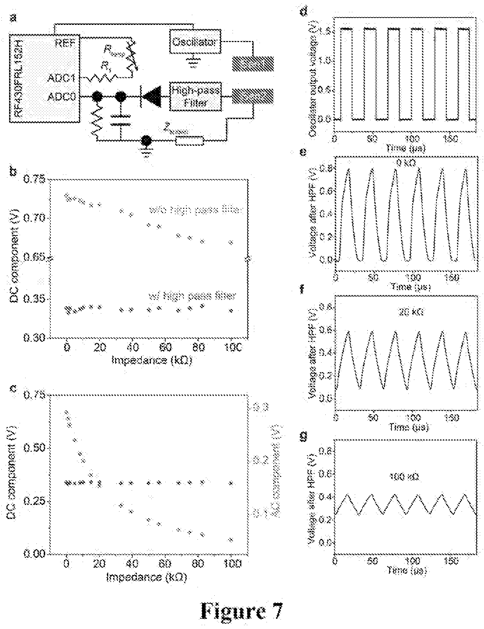

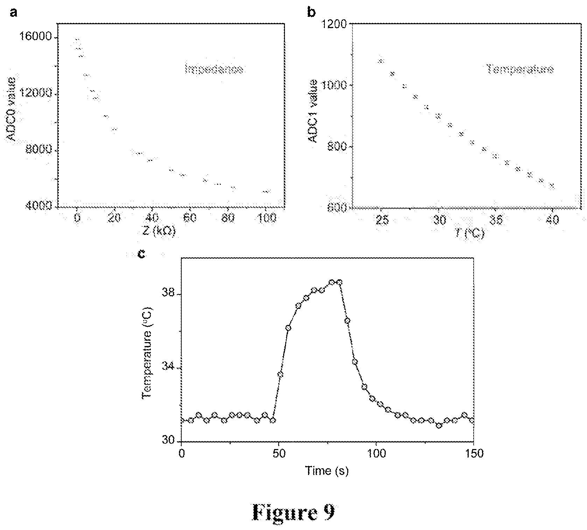

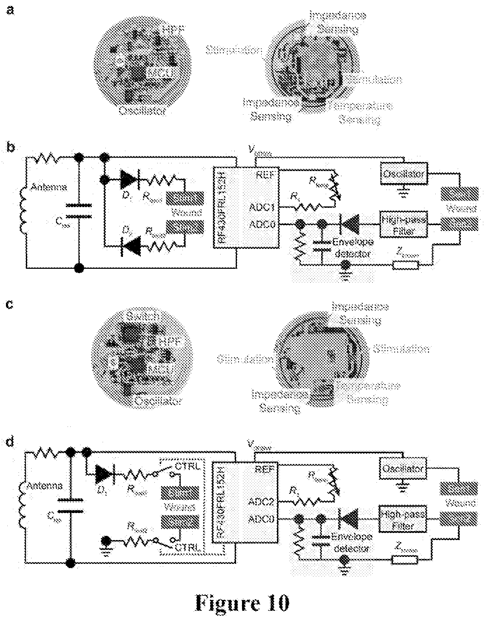

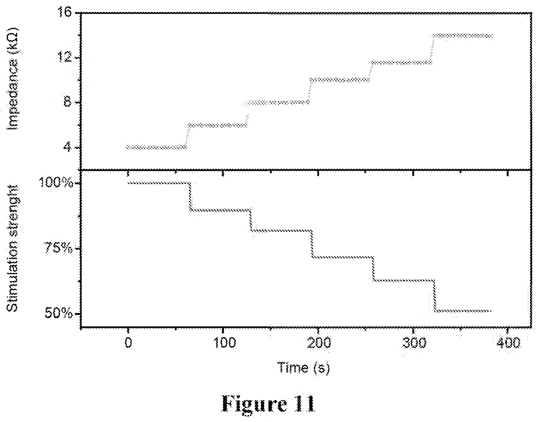

The NFC transponder of example embodiments (e.g. RF430FRL152H shown in Figure 2a) offers two 14-bits analog-digital converters (ADCs) to serve as the analog front-end interface. To best monitor the condition of the wound, embodiments integrate two sensors (one thermistor and one impedance sensor) in sensor 202, which serve as good proxies for determining infection and inflammatory states of the wound (Power, G., Moore, Z. & O'Connor, T. Measurement of pH, exudate composition and temperature in wound healing: a systematic review. J Wound Care 26, 381-397, doi:10.12968/jowc.2017.26.7.381 (2017)). The RF430FRL152H transponder has a direct thermistor support (ADC1 channel) by emitting a small µA level current on the thermistor and sampling the voltage. For impedance sensing, an oscillator was used to generate a 32.768 kHz square wave alternating current (AC) signal (Fig. 7) that passed through the wound, and a known impedance component (Zknown). Through a voltage divider, the AC signal applied on Zknown could then reflect the wound impedance as shown in Figure 2f and Figure 7. This received AC signal is further conditioned through a high-pass filter to remove the random direct current (DC) component inside the oscillation signal as shown in Figure 2g and Figure 7. Finally, an envelope detector is used to convert the AC signal amplitude to a DC voltage, which is captured by the ADC0 channel inside the RF430FRL152H transponder. With standard impedance components and controlled temperature, both ADC channels are calibrated in the integrated design as shown in Figures 2h, 2i and 9-10. Finally, the sensing data from sensors 202 can be analyzed in real-time to provide feedback on the stimulation pattern for closed-loop operation as shown in Figure 11).

### Hydrogel interface

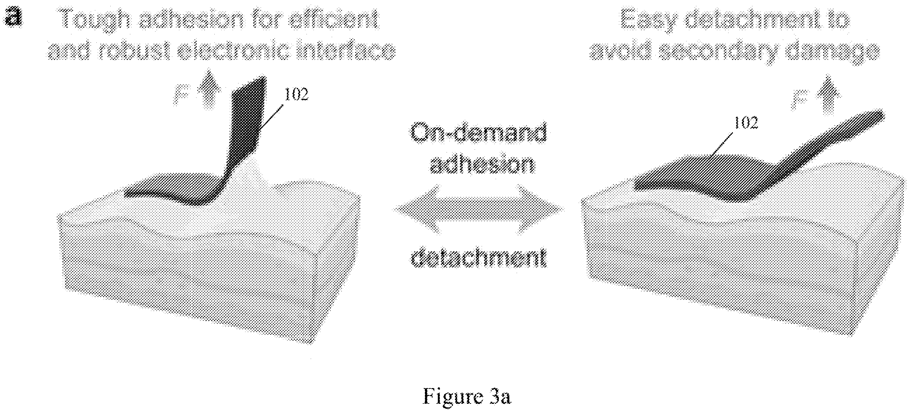

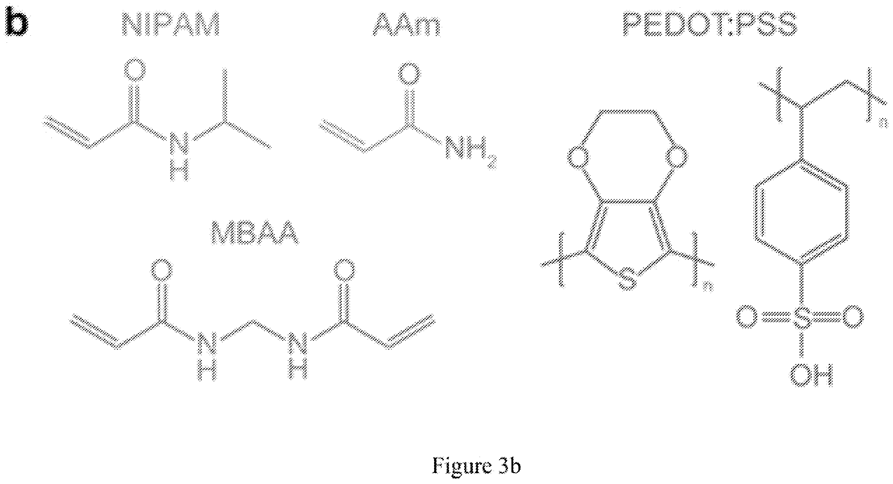

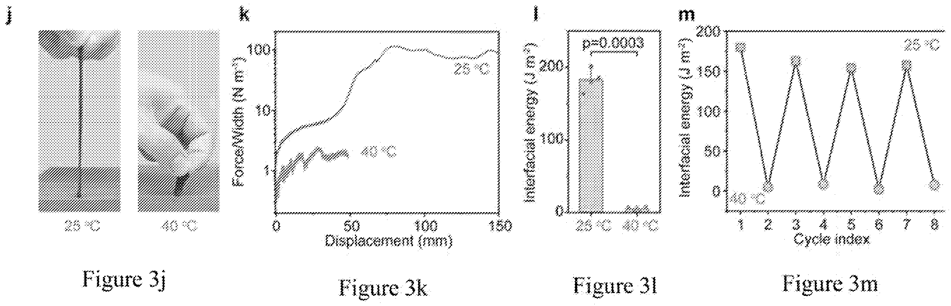

Figures 3a to 3m illustrate example aspects of a tough and low-impedance conductive hydrogel electrode with reversible tissue adhesion according to embodiments. Figure 3a is a functional diagram illustrating the requirements for the hydrogel interface in the smart bandage. As shown, during device operation (left), the hydrogel electrode needs to possess simultaneously high toughness and adhesion to avoid damage or detachment. When peeling off the device after the treatment period (right), the tissue interfacing gel needs to be easily detachable to minimize secondary damage to the delicate wounded tissue. Figure 3b is a diagram illustrating example Molecular structures of the monomers, crosslinker, and conducting polymer for the interpenetrated double network.

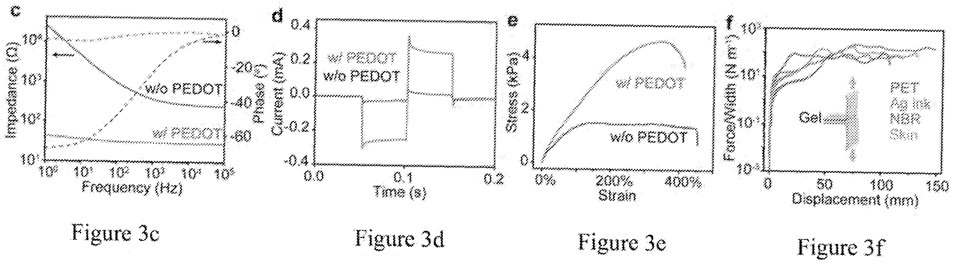

Figures 3c and 3d are graphs illustrating electrochemical impedance spectroscopy (EIS) and chronoamperometry, respectively, of example hydrogels according to embodiments with (20 mg mL⁻¹ PEDOT:PSS with 150 mg mL⁻¹ NIPAM and 12 mg mL⁻¹ AAm) and without (150 mg mL⁻¹ NIPAM and 12 mg mL⁻¹ AAm only) PEDOT:PSS. Figure 3e is a graph illustrating, Uni-directional tensile tests of example hydrogels with and without PEDOT:PSS. Figure 3f is a graph illustrating a 180° peeling test of an example conducting hydrogel according to embodiments on various surfaces including polyethylene terephthalate (PET), screen printed and dried Ag ink, nitrile butadiene rubber (NBR), and mouse skin tissue.

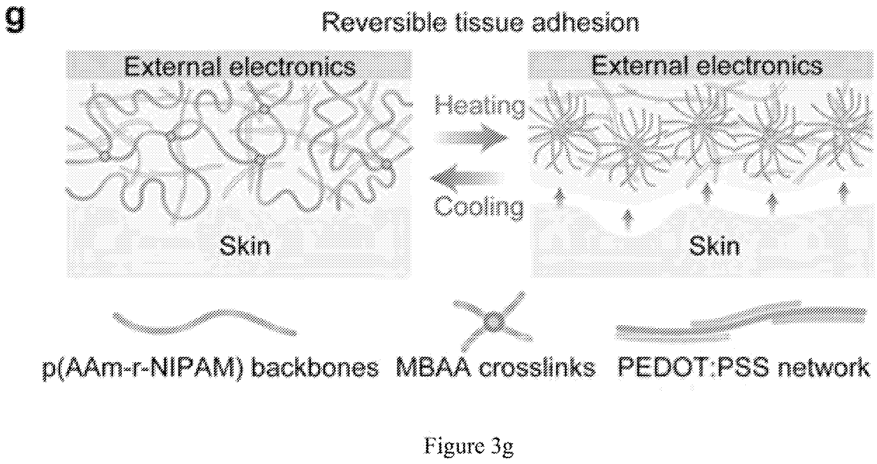

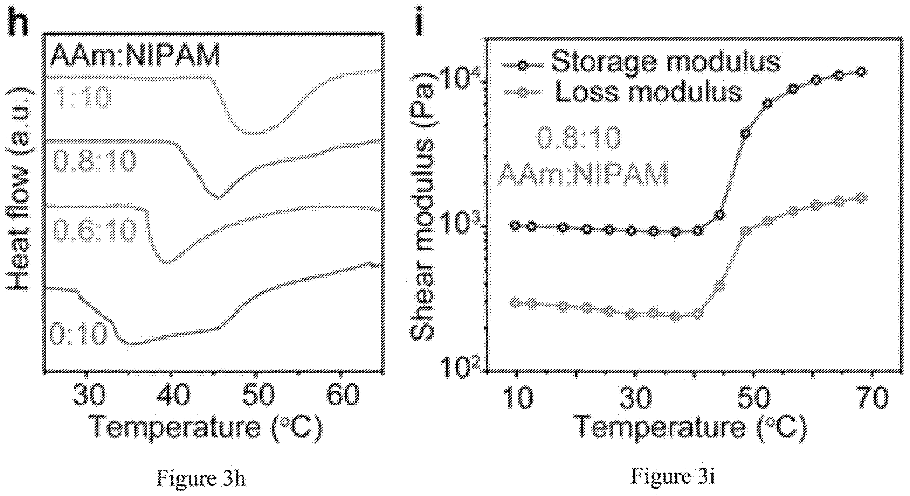

Figure 3g is a functional diagram illustrating the microscopic structural changes during the lower critical solution temperature (LCST) phase transition of an example hydrogel according to embodiments. Figure 3h is a graph illustrating differential scanning calorimetry (DSC) scans of an example hydrogel interface with different acrylamide (AAm) to N-isopropylacrylamide (NIPAM) weight ratios. The hydrogel in this example consists of 150 mg mL⁻¹ NIPAM, 20 mg mL⁻¹ PEDOT:PSS, and AAm of 0, 9, 12, and 15 mg mL⁻¹. Figure 3i is a graph illustrating a rheological measurement showing the phase transition temperature of the sample when the AAm to NIPAM weight ratio is 0.8:10.

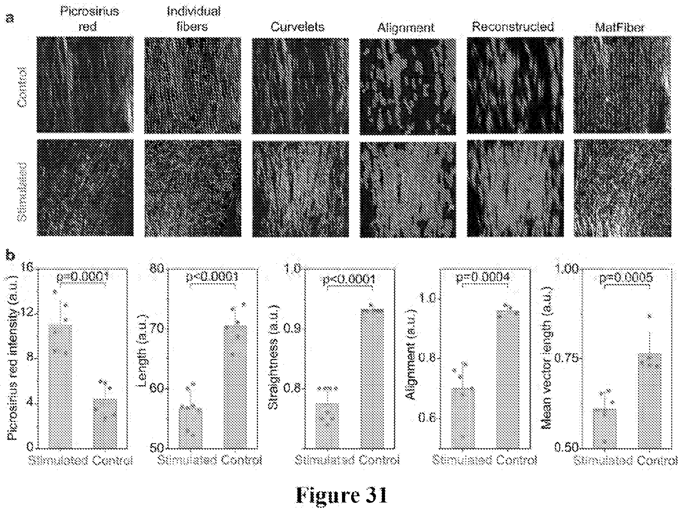

Figure 3j are photographs and Figure 3k is a graph illustrating a 180 °C peeling test of an example hydrogel according to embodiments. Figure 31 is a graph illustrating the drastic differences in adhesion for the gel at room temperature and 40 °C. The tunable hydrogel adhesion can be cycled for multiple times as shown in Figure 3m due to the reversible nature of the LCST phenomenon. The hydrogel in the example of Figures 3j-3m consists of 150 mg mL⁻¹ NIPAM, 12 mg mL⁻¹ AAm, and 20 mg mL⁻¹ PEDOT:PSS.

As can be seen from above, to ensure an intimate skin interface and robust electrical communication between the circuit and tissue through the soft hydrogel, the gel electrode interface should have the following characteristics: low contact impedance, high toughness, and tunable adhesion. The low contact impedance is preferred so as to ensure sensitive sensing and efficient charge injection by electrical stimulation. The high toughness requirement is preferred so as to avoid mechanical damage during motion. Finally, the tissue interfacing gel should preferably have on-demand adhesion to the wound tissue to provide good adhesion during therapy while also easy, gentle removal upon external triggers (e.g., gentle heating) to mitigate secondary damage to the delicate wounded tissue and prevent a commonly occurred skin condition known as medical adhesive-related skin injury (Kelly-O'Flynn, S., Mohamud, L. & Copson, D. Medical adhesive-related skin injury. Br J Nurs 29, S20-S26, doi:10.12968/bjon.2020.29.6.S20 (2020); Fumarola, S. et al. Overlooked and underestimated: medical adhesive-related skin injuries. J Wound Care 29, S1-S24, doi:10.12968/jowc.2020.29.Sup3c.S1 (2020)).

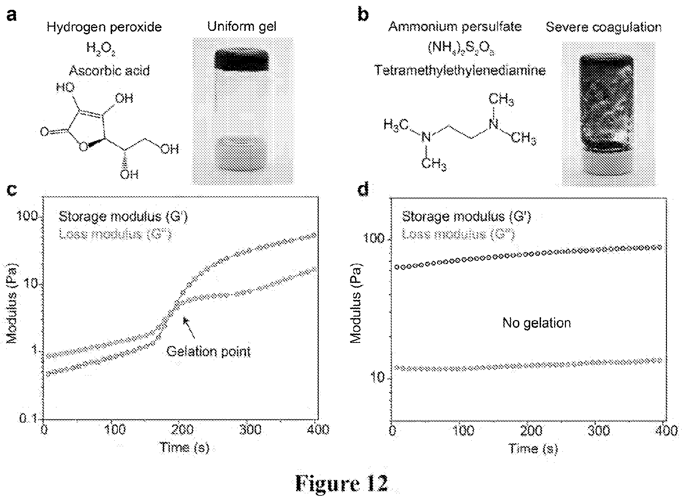

As shown in Figure 3b, the present embodiments provide an inter-penetrated double-network structure through in situ radical polymerization of a thermal-responsive covalent network of N-isopropylacrylamide (NIPAM) (Schild, H. G. Poly(N-isopropylacrylamide): experiment, theory and application. Progress in Polymer Science 17, 163-249, doi:10.1016/0079-6700(92)90023-r (1992)) and acrylamide (AAm) in the presence of a physically crosslinked conducting polymer network of PEDOT:PSS. Notably, since PEDOT:PSS exists in the form of a colloidal aqueous suspension, it would severely coagulate when mixed with conventional radical initiators that contain both ionic and basic species, i.e., ammonium persulfate (AP) and N,N,N',N'-tetramethylethylenediamine (TEMED) (Figure 12). To ensure uniform gel formation, a new initiation system is included based on a non-ionic redox pair of hydrogen peroxide and ascorbic acid, that allows for rapid and homogeneous gelation at room temperature (~3 min) (Figure 12).

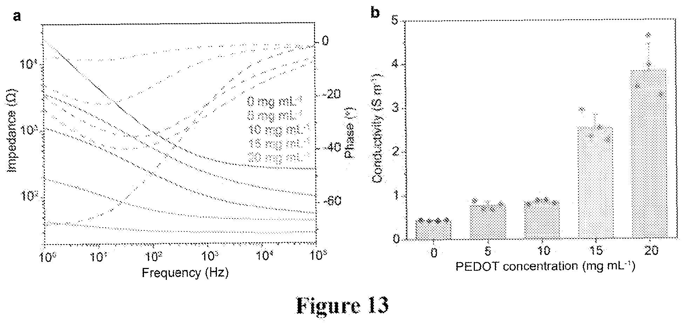

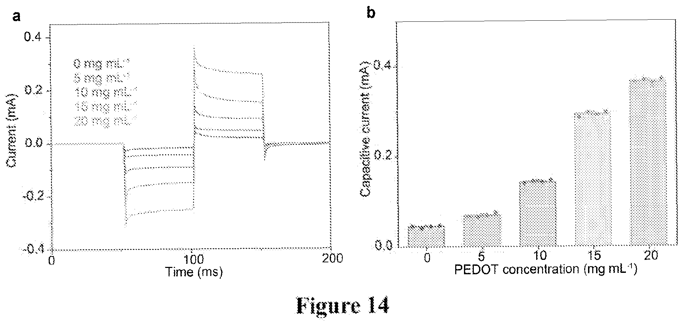

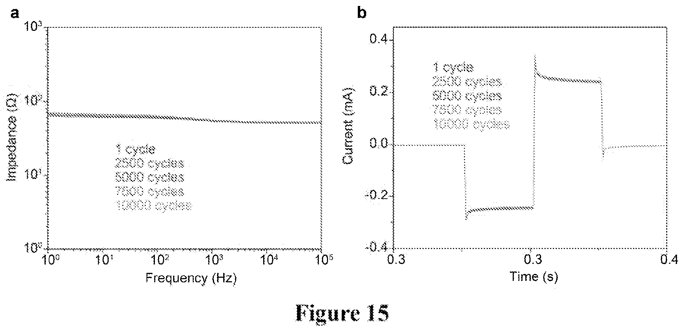

Compared to the pristine poly(NIPAM-ran-AAm) gel, the incorporation of PEDOT:PSS substantially reduced the interfacial impedance when in contact with phosphate buffered saline (PBS) with a ~0° phase angle across the entire frequency range as shown in Figure 3c and Figure 13, corresponding to a resistive nature for the contact due to the high capacitance at low frequency range for PEDOT:PSS35. Similarly, when a voltage pulse was applied, the PEDOT:PSS gel showed substantially enhanced charge injection capacity when compared to the control sample as shown in Figure 3d and Figure 14, which ensures efficient delivery of stimulus from the electronically conducting circuits to ionically conducting tissues. The low impedance and high charge injection of the hydrogel electrode can be well maintained even after 10,000 cycles of repetitive charge injections (Figure 15).

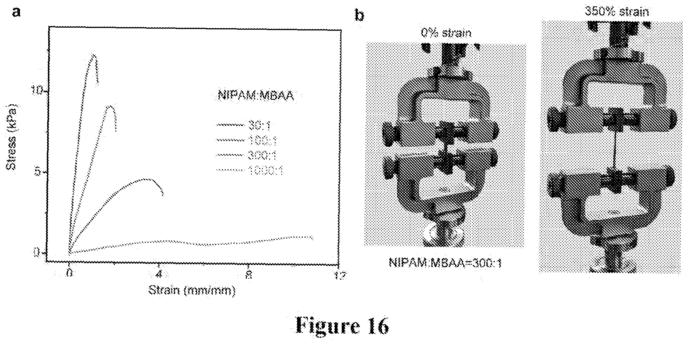

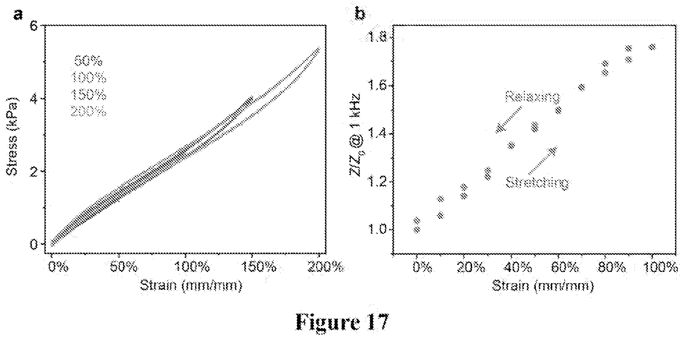

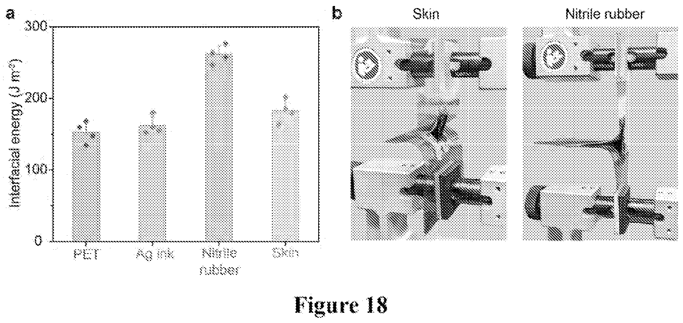

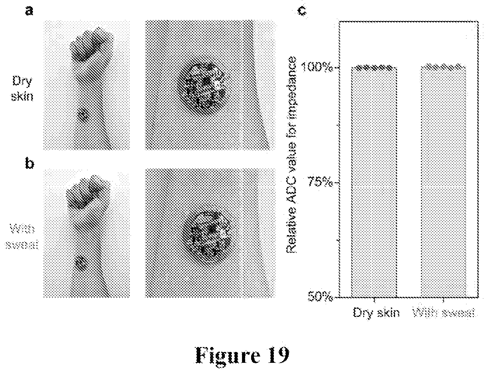

In addition to improved electrical performances, the incorporation of PEDOT:PSS also enhanced the mechanical properties of the hydrogel. Under a uni-directional tensile test, the composite gel can be stretched to a similar strain as the control poly-NIPAM gel (~400%) but with a higher Young's modulus, giving rise to a higher toughness as shown in Figure 3e and Figure 16. The composite hydrogel is elastic with reversible impedance changes upon stretching to at least 100% strain (Figure 17). Finally, because of the high content of polar moieties in the NIPAM-AAm backbone, the composite hydrogel can have polar interactions in addition to van der Waals interactions with diverse surfaces, such as plastic, metal, rubber, or skin, to give its strong interfacial adhesion (Figure 3f, Figures 18-19).

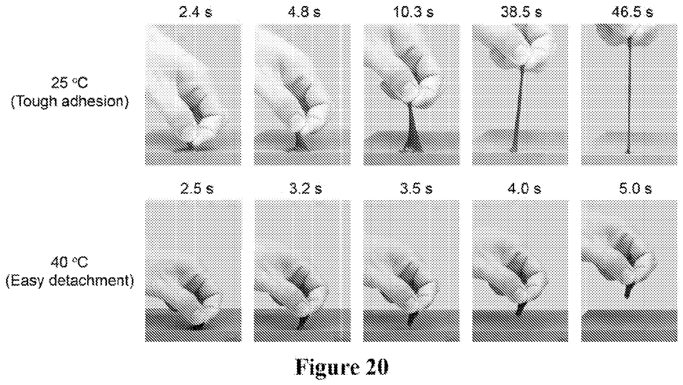

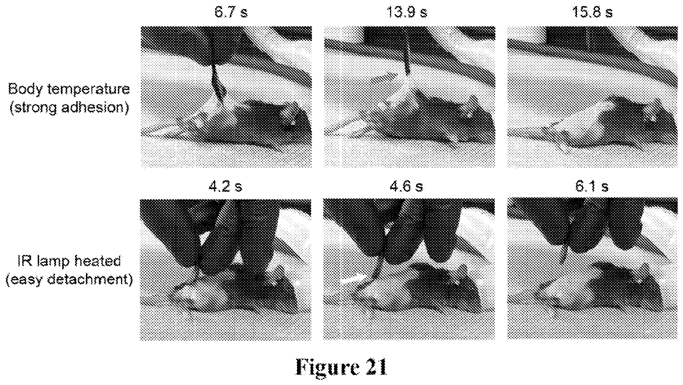

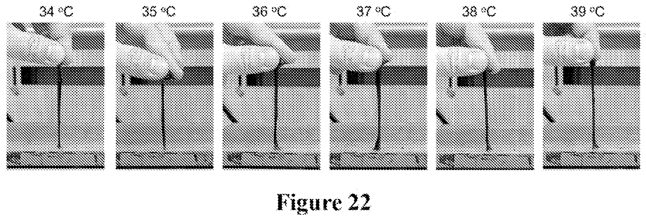

Although hydrogels containing NIPAM and PEDOT:PSS have been previously studied (Cao, S., Tong, X., Dai, K. & Xu, Q. A super-stretchable and tough functionalized boron nitride/PEDOT:PSS/poly(N-isopropylacrylamide) hydrogel with self-healing, adhesion, conductive and photothermal activity," Journal of Materials Chemistry A 7, 8204-8209, doi:10.1039/c9ta00618d (2019)), a dual-conducting hydrogel with on-demand tissue adhesion and detachment has not been reported. Poly-NIPAM is a well-known polymer that exhibits a lower critical solution temperature (LCST) in water due to the heat induced aggregation of the amphiphilic NIPAM units. In the present case, it was observed that the LCST transition was associated with drastic changes in gel adhesion, likely because the aggregated backbones can no longer form effective bonding sites with external surfaces as shown in Figure 3g. It was found that additional hydrophilic monomers of AAm can be used to tune the LCST point to higher levels (i.e., above body temperature) (Fundueanu, G., Constantin, M. & Ascenzi, P. Poly(N-isopropylacrylamide-co-acrylamide) cross-linked thermoresponsive microspheres obtained from preformed polymers: Influence of the physico-chemical characteristics of drugs on their release profiles. Acta Biomater 5, 363-373, doi:10.1016/j.actbio.2008.07.011 (2009)), as indicated from differential scanning calorimetry (DSC) (Fig. 3h). When the mass ratio between AAm and NIPAM monomers was 0.8:10, the phase change temperature reached ~40 °C, as confirmed by both DSC and rheological measurements as shown in Figures 3h and 3i. When tested on metal and mouse skin, the hydrogel electrodes showed strong adhesion at room temperature or normal skin temperature, comparable to 3MTM Kind Removal Silicone Tape used to secure gauze to the skin, but completely lost its adhesion with two orders of magnitude lower interfacial energy when heated above 40 °C as shown in Figures 3j-3l and Figures 20-21. Of note, the phase transition will not occur gradually before the critical temperature, as evidenced by DSC and rheology as shown in Figures 3h and 3i, which prevents undesired detachment during normal operation (Figure 22). Finally, because the LCST process is reversible (Zhang, Q., Weber, C., Schubert, U. S. & Hoogenboom, R. Thermoresponsive polymers with lower critical solution temperature: from fundamental aspects and measuring techniques to recommended turbidimetry conditions. Materials Horizons 4, 109-116, doi:10.1039/c7mh00016b (2017)), the tunable adhesion of the same hydrogel can be repeated multiple times without significant degradation of the low-temperature adhesion as shown in Figure 3m.

### Validation in pre-clinical wound models

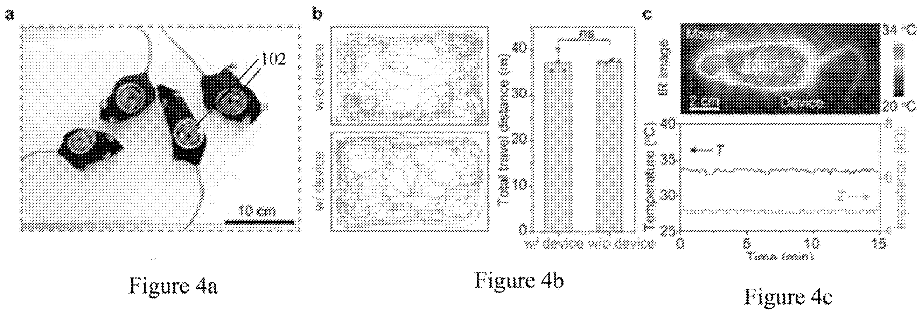

Figures 4a to 4l illustrate aspects of how an example wireless smart bandage system according to embodiments can continuously monitor physiological wound conditions and accelerate tissue regeneration. Figure 4a is a Photograph of four freely-moving mice wearing wireless smart bandages according to embodiments. Figure 4b are graphs illustrating representative trajectories of mice with and without the smart bandage in the open-field test (left), and a statistical analysis showing no significant differences between two groups (right). Four mice in each group were used in the test. Figure 4c is an Infrared (IR) image of a mouse wearing the smart bandage of embodiments (upper) and raw traces of wirelessly sensed wound temperature and impedance (lower).

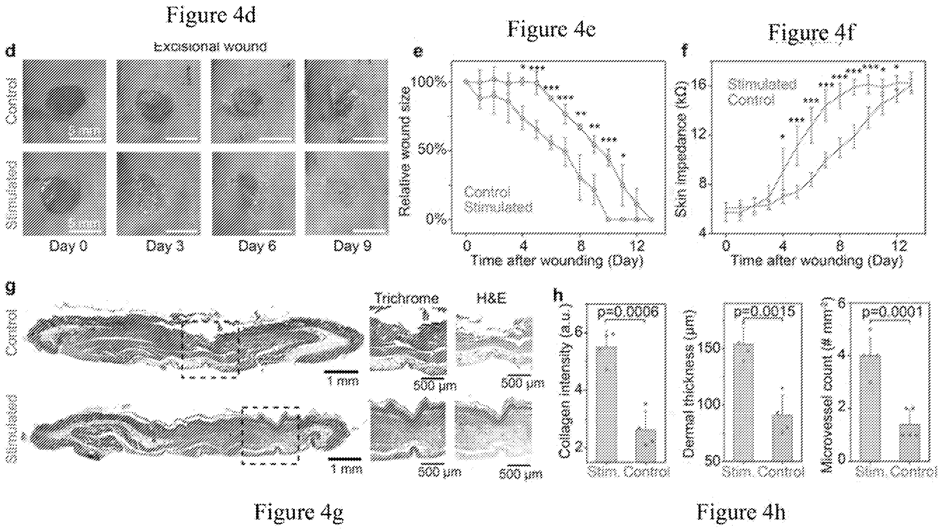

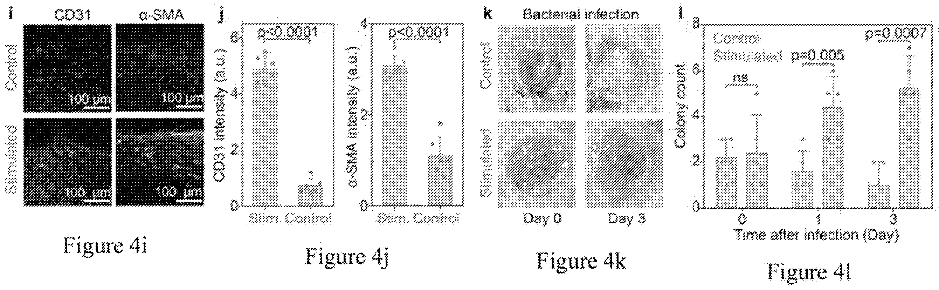

Figure 4d are representative photos showing the progression of wound regeneration in an excisional wound healing model with (lower) and without (upper) electrical stimulation treatment. Figures 4e and 4f are graphs illustrating the relative size and impedance, respectively, of excisional wounds over time, indicating accelerated tissue regeneration with stimulation. N=5 for each group. All data are represented as mean ± standard deviation. In the figures, a two-tailed t-test assuming equal variances were performed for the p values. * denotes p < 0.05, ** denotes p < 0.01, *** denotes p < 0.001. Figure 4g provides representative cross-sectional histology images of skin tissue harvested from mice with (lower) and without (upper) stimulation after 13 days. The left and middle images illustrate Masson's trichrome; the right images illustrate hematoxylin and eosin (H&E). The black dashed boxes mark the area for zoomed-in views in the middle and right panels highlighting the healed tissue. Visible intact epidermal and dermal layer observed in stimulated treatment group in the Masson's Trichrome stain, visualized by a red surface layer which stains for muscle cells and blue layer below, which stains for collagen. Figure 4h are three graphs illustrating a Quantitative comparisons of collagen intensity, dermal thickness, and microvessel count, respectively, for skin tissue with and without stimulation. All data are represented as mean ± standard deviation. Two-tailed t-test assuming equal variances were performed. Figures 4i and 4j are Immunostaining images and quantitative comparisons, respectively, of CD31 and α-SMA from tissue with and without stimulation. Figures 4k and 4l are Representative photos and quantitative comparisons, respectively, of wounds infected with E. coli, with and without stimulation. All data are represented as mean ± standard deviation. Two-tailed t-test assuming equal variances were performed.

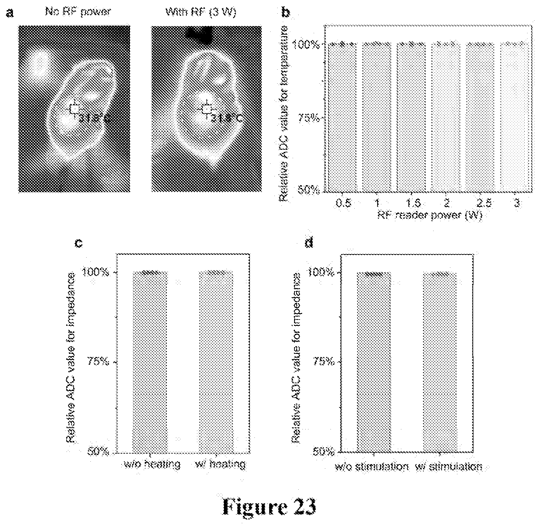

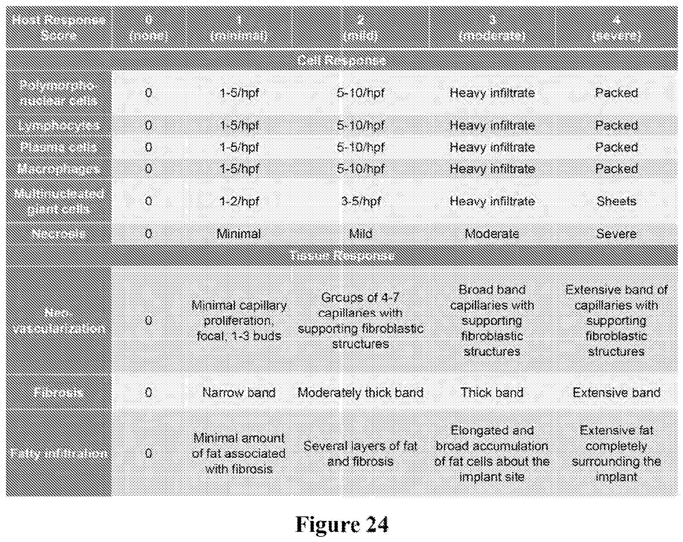

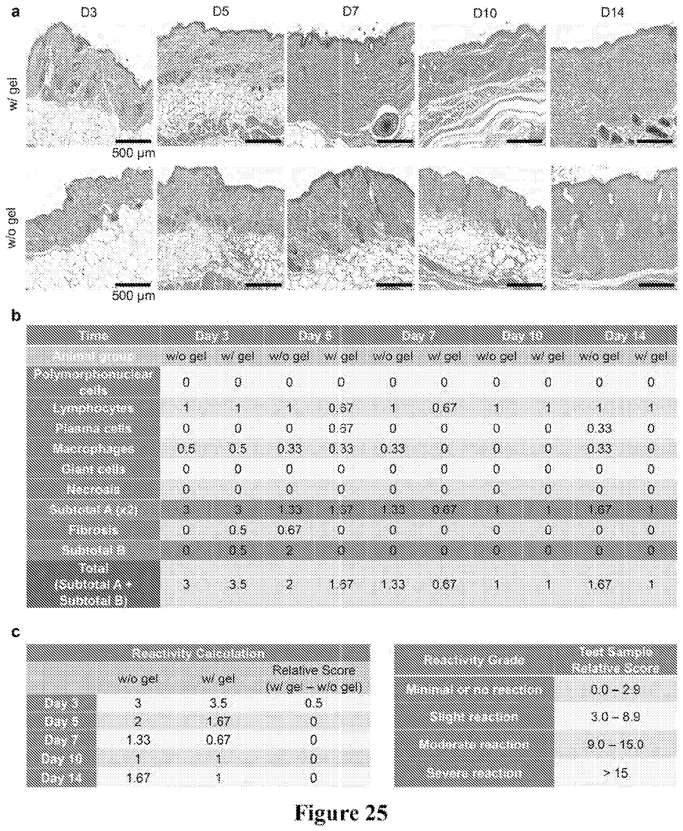

As shown in the above, figures, to validate an example wound care management system of embodiments, a series of pre-clinical evaluations were performed to test the robustness and efficacy of the device in its ability to monitor wound conditions and deliver timely treatment. First it was confirmed that mice wearing the wireless devices were able to move freely with a similar distance traveled as mice with no device attached, demonstrating an ideal therapeutic modality for patient use: namely lightweight and untethered with cables as shown in Figures 4a and 4b. More importantly, the temperature and impedance sensors were able to monitor the state of the wound continuously as the mice moved freely in the cage as shown in Figures 4c and Figure 23. In addition, the hydrogel was biocompatible and did not initiate any sensitization or irritation after continuous contact with the skin for 15 days, demonstrating no adverse reactivity signs compared to normal skin (Figure 25, Figure 24).

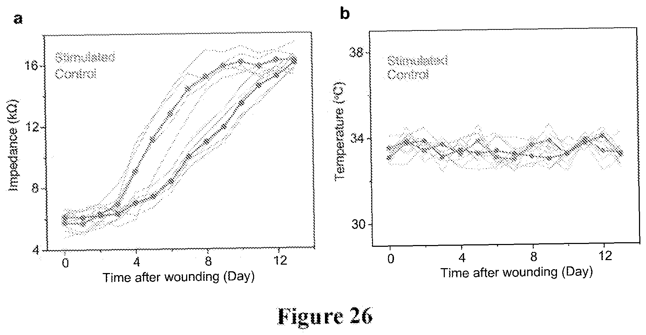

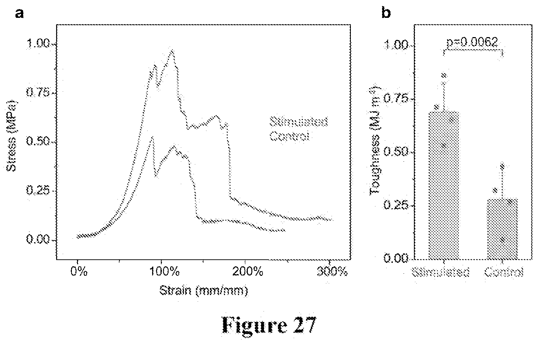

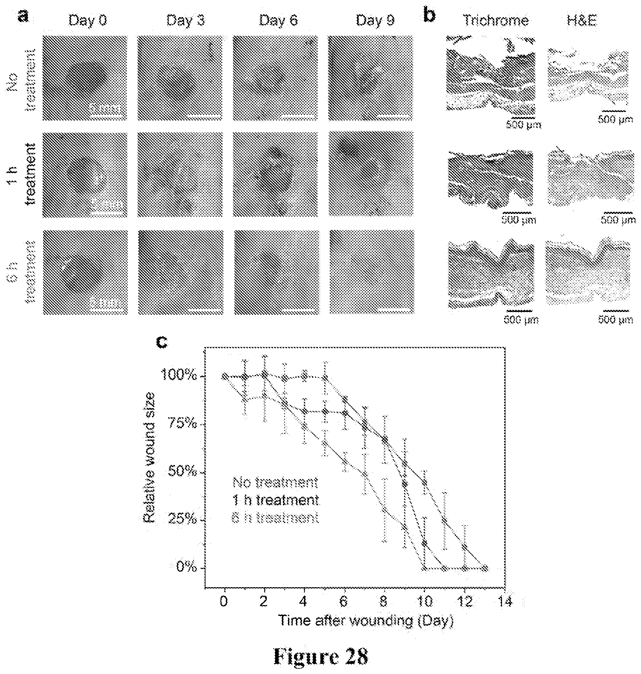

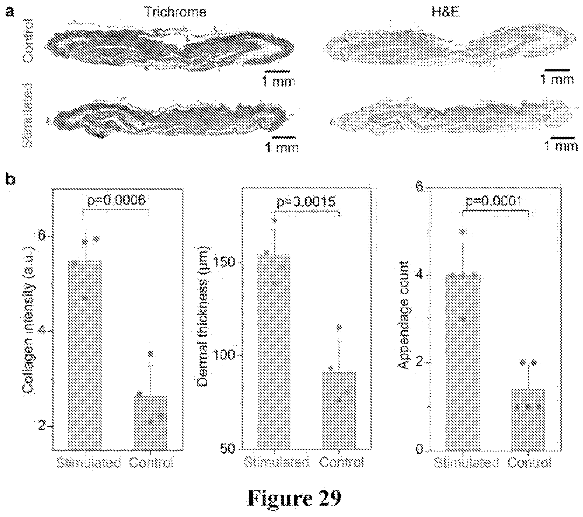

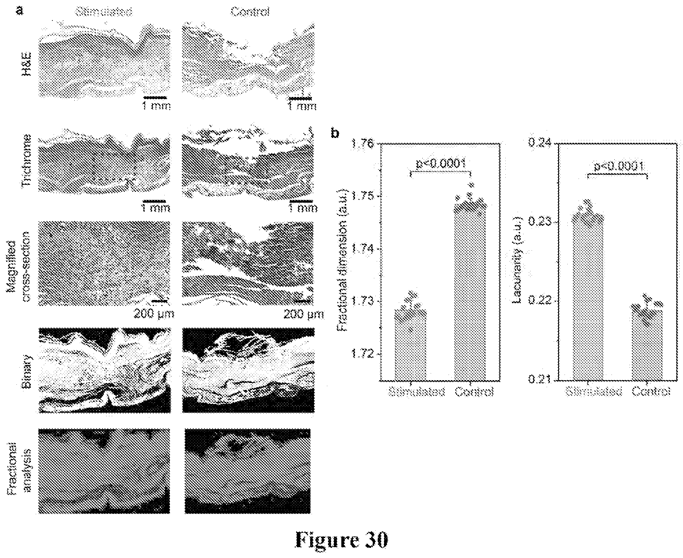

To test the functionality of an example platform of embodiments in a biological system, a splinted excisional wound mouse model was used, where stimulated mice were treated with continuous electrical pulses. Control mice received standard sterile wound dressings without electrical stimulation. It was found that stimulation resulted in accelerated wound closure as shown in Figures 4d and 4e and a significant increase in wound impedance to attain a faster impedance plateau, signifying a return to an unwounded state as shown in Figures 4f and Figure 26. Stimulation of wounds also improved functional tensile recovery with increased dermal thickness, collagen deposition and overall dermal appendage count as shown in Figures 4g and 4h and Figures 27-29. Of note, compared to a wired modality, the present smart bandage allowed for longer and potentially continuous treatment durations (Figure 28), which have been linked to accelerated wound closures. Stimulated wounds also showed an increase in the collagen fiber heterogeneity, resulting in more random, shorter, and less aligned fiber orientations (Figures 30-31).

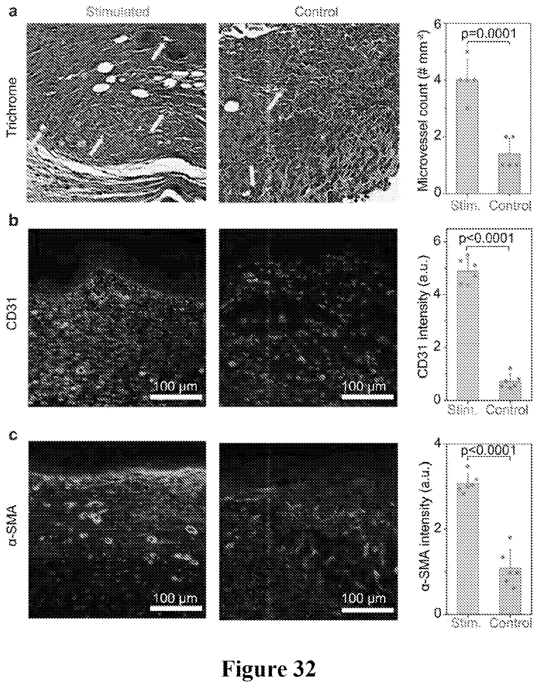

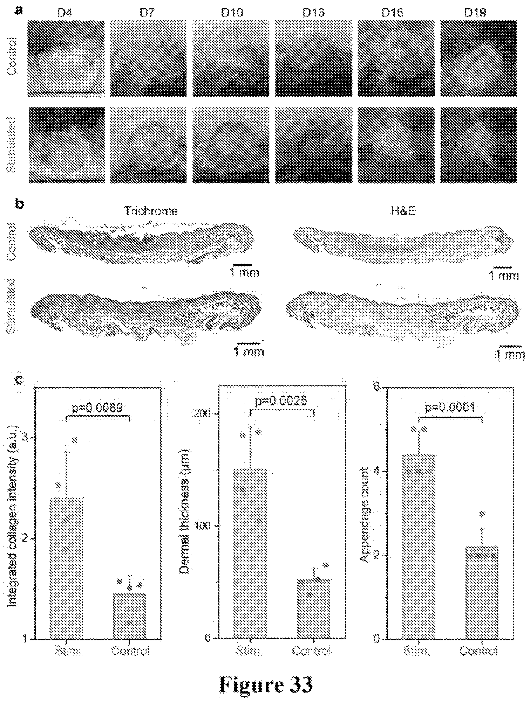

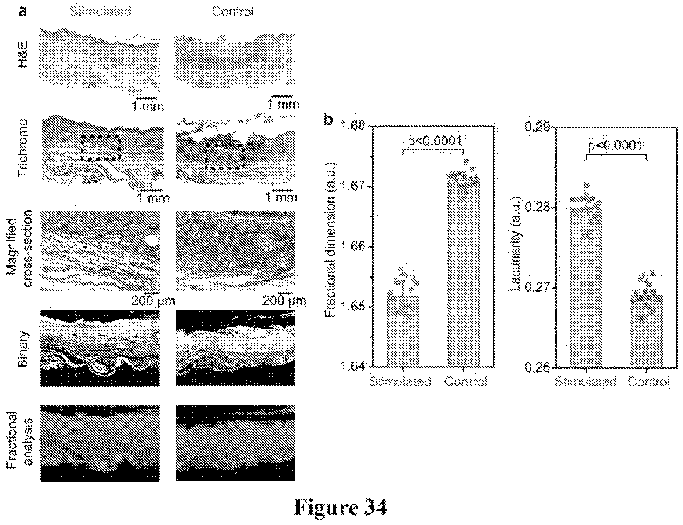

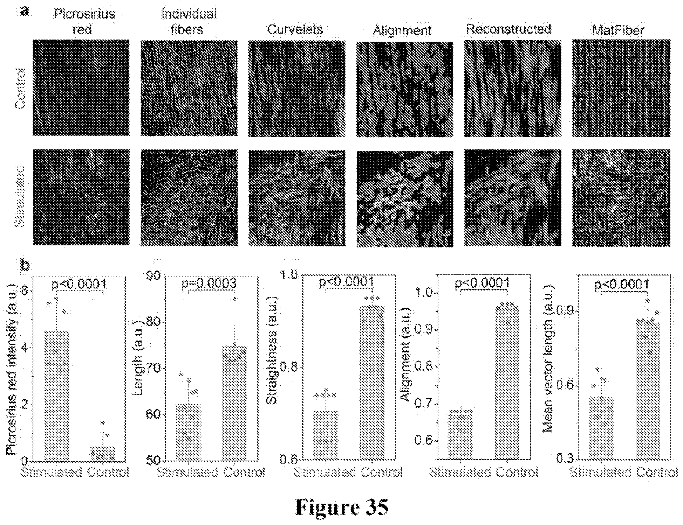

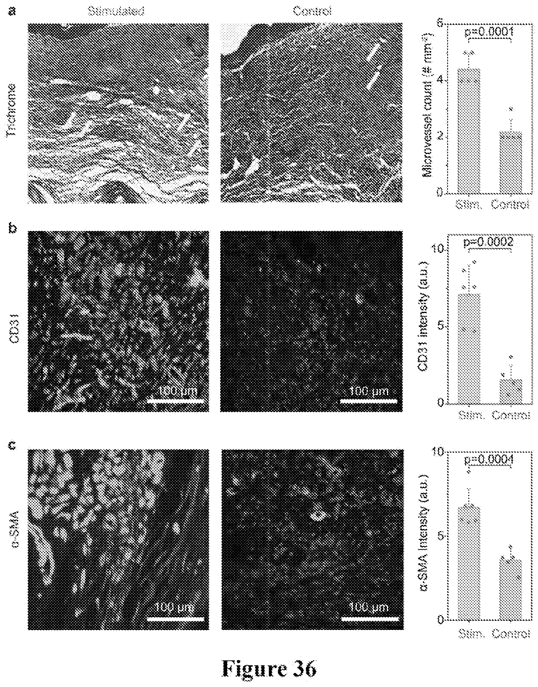

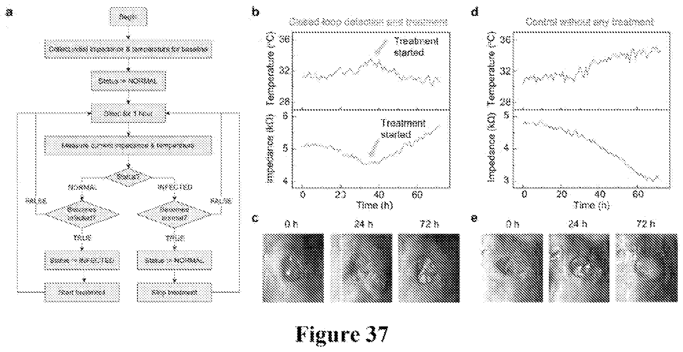

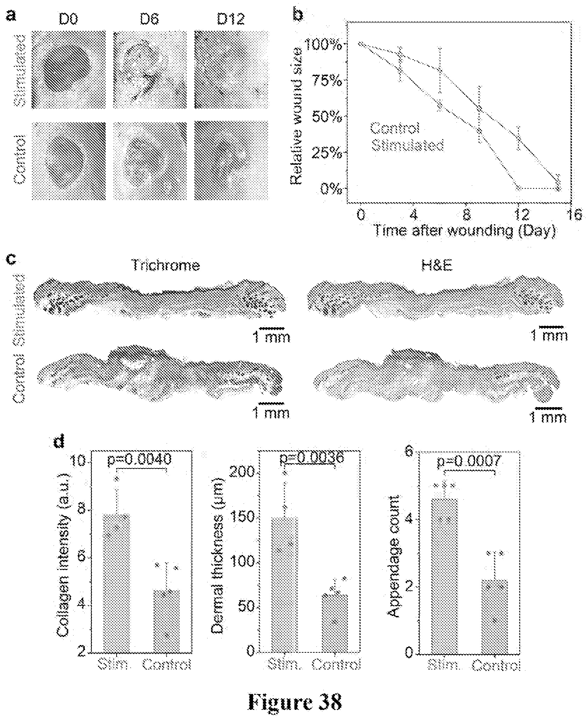

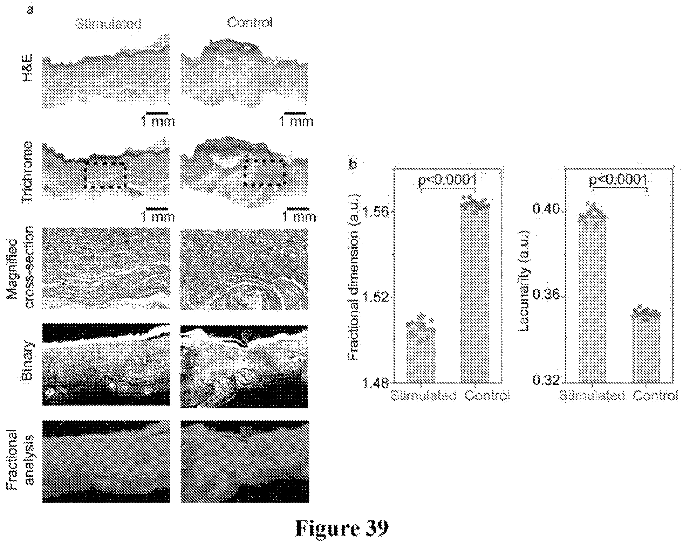

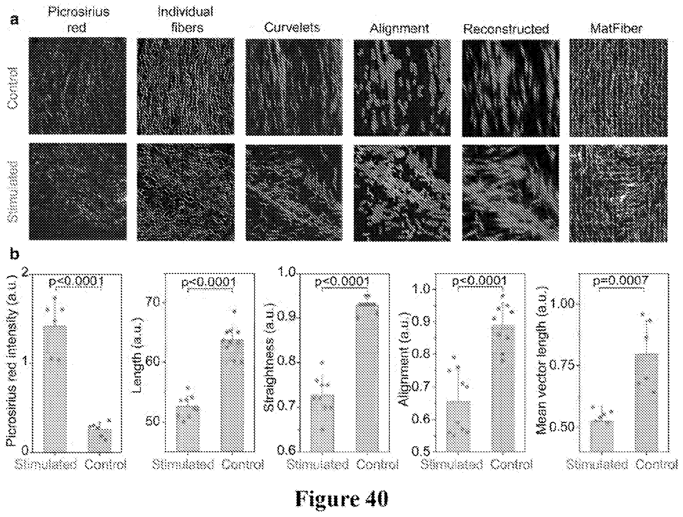

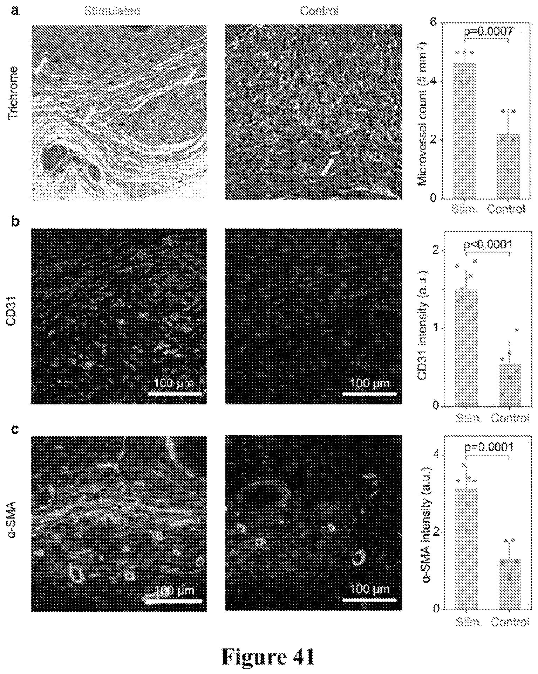

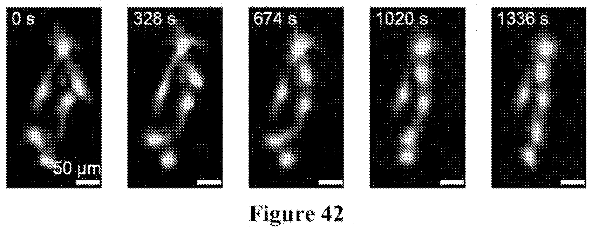

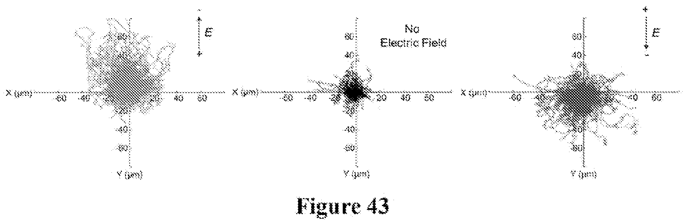

Further observed was a significant increase in neovascularization among stimulated wounds, with an increased microvessel count and higher CD31 and α-SMA expression as shown in Figures 4h-4j, Figure 32. Similar results were also observed in a murine burn wound healing model (Figures 33-36). The present smart bandage was also found to significantly reduce infection in the wound, decreasing overall bacterial colony count as shown in Figures 4k-4l. Moreover, by continuously monitoring wound impedance and temperature, the present wireless smart bandage could detect early onset of infection and modulate treatment in a closed-loop manner to avoid further wound complications (Figure 37). In current clinical practices, doctors still rely on qualitative markers such as swelling or erythema (i.e., superficial reddening of the skin) to identify wound infections, which are often difficult to judge in the early stages of biofilm development. With quantitative biophysical signals recorded by the present smart bandage, one can provide treatment when clinically-used markers are still ambiguous, enabling timely treatment of chronic wounds, reduce hospital readmissions and medical cost, and improve patient wound healing outcomes (Negut, I., Grumezescu, V. & Grumezescu, A. M. Treatment Strategies for Infected Wounds. Molecules 23, doi:10.3390/molecules23092392 (2018)). We further validated our system in a streptozotocin (STZ) induced diabetic excisional wound model (Chen, H. et al. Dissolved oxygen from microalgae-gel patch promotes chronic wound healing in diabetes. Sci Adv 6, eaba4311, doi:10.1126/sciadv.aba4311 (2020)), also observing an accelerated time to wound closure, improved dermal collagen fiber heterogeneity, and increased vascularization (Figures 38-41). An STZ model most closely resembles Type I diabetes in patients (Wu, J. & Yan, L. J. Streptozotocin-induced type 1 diabetes in rodents as a model for studying mitochondrial mechanisms of diabetic β cell glucotoxicity. Diabetes Metab Syndr Obes 8, 181-188, doi:10.2147/DMSO.S82272 (2015)). On the cellular level, observed was the expected ability of the present device to prompt cell alignment and migration, inducible with a directional electric field (Figures 42-43).

### Cellular and Molecular Mechanism

Although the beneficial effects of electrical stimulation have been previously reported, the cellular and molecular mechanisms for this effect remain obscure. Previous works have evaluated the role of electrical stimulation in enhancing wound healing through the activation of fibroblasts and keratinocytes, both known major cell types of the dermis that are active in the inflammatory phase of cutaneous wound repair (Schutzius, G. et al. BET bromodomain inhibitors regulate keratinocyte plasticity. Nat Chem Biol 17, 280-290, doi:10.1038/s41589-020-00716-z (2021); Mahmoudi, S. et al. Heterogeneity in old fibroblasts is linked to variability in reprogramming and wound healing. Nature 574, 553-558, doi:10.1038/s41586-019-1658-5 (2019); Chen, K. et al. Disrupting biological sensors of force promotes tissue regeneration in large organisms. Nat Commun 12, 5256, doi:10.1038/s41467-021-25410-z (2021); Trotsyuk, A. A. et al. Inhibiting Fibroblast Mechanotransduction Modulates Severity of Idiopathic Pulmonary Fibrosis. Adv Wound Care (New Rochelle), doi:10.1089/wound.2021.0077 (2021); Barrera, J. A. et al. Adipose-Derived Stromal Cells Seeded in Pullulan-Collagen Hydrogels Improve Healing in Murine Burns. Tissue Eng Part A 27, 844-856, doi:10.1089/ten.TEA.2020.0320 (2021)). Inflammatory signals activate the maturation and cross talk between these two cell types, coordinating the migration and restoration of normal tissue homeostasis after wounding (Barrientos, S., Stojadinovic, O., Golinko, M. S., Brem, H. & Tomic-Canic, M. Growth factors and cytokines in wound healing. Wound Repair Regen 16, 585-601, doi:10.1111/j.1524-475X.2008.00410.x (2008); Chen, K. et al. Mechanical Strain Drives Myeloid Cell Differentiation Toward Proinflammatory Subpopulations. Adv Wound Care (New Rochelle), doi:10.1089/wound.2021.0036 (2021)). However, the effect of electrical stimulation on immune cells, namely circulating cells, which are critical regulators of all stages of wound healing from early inflammation until late fibrosis (Kim, S. Y. & Nair, M. G. Macrophages in wound healing: activation and plasticity. Immunol Cell Biol 97, 258-267, doi:10.1111/imcb.12236 (2019); Wynn, T. A. & Vannella, K. M. Macrophages in Tissue Repair, Regeneration, and Fibrosis. Immunity 44, 450-462, doi:10.1016/j.immuni.2016.02.015 (2016)), remains unexplored.

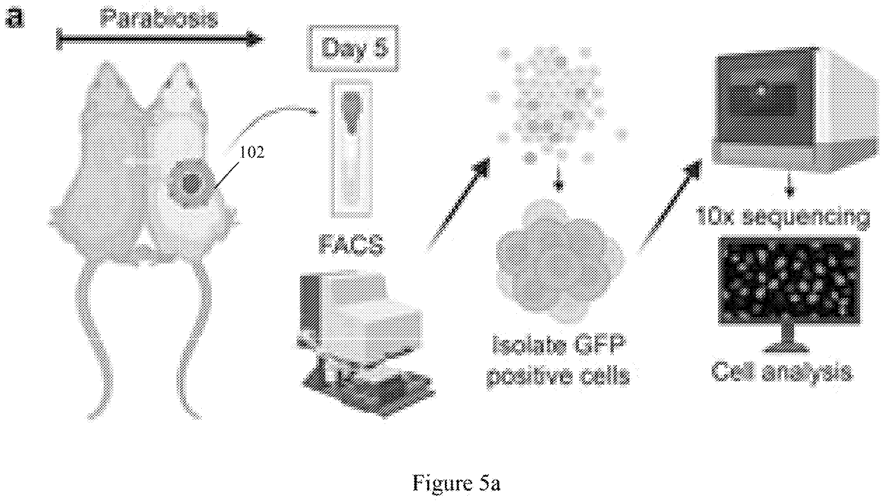

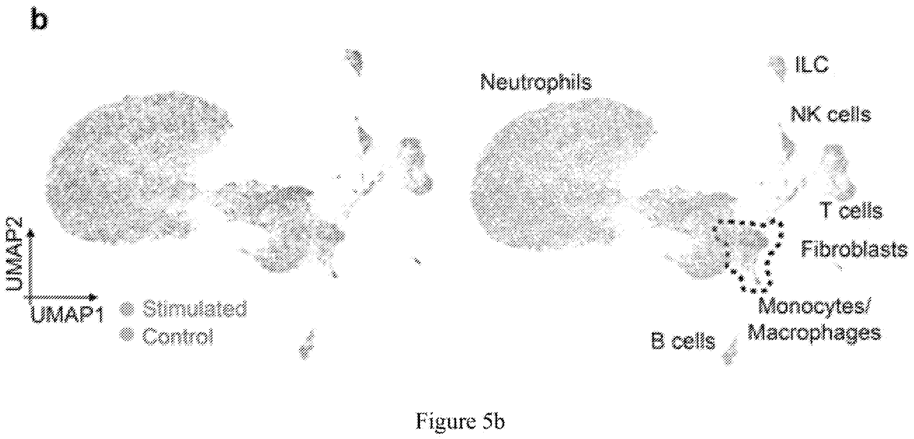

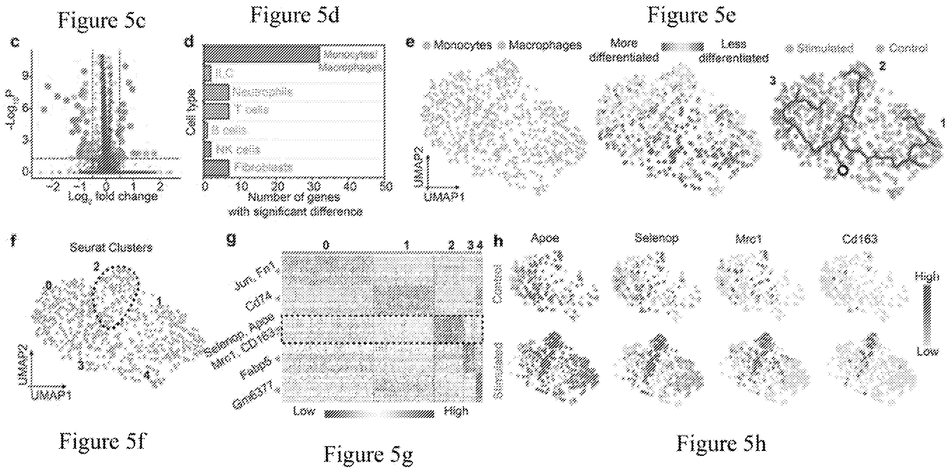

Figures 5a to 5k illustrate aspects of the molecular mechanism attributing to the accelerated tissue regeneration with electrical stimulation according to embodiments. Figure 5a is a functional diagram illustrating the experimental flow for an example single-cell RNA sequencing (scRNA-seq). Tissues from an excisional wound of a wild type (WT) mouse paired with a GFP-positive mouse, subjected to either treatment (i.e., stimulation) or not (i.e., control), were sorted for GFP-positive cells using fluorescent activated cell sorting (FACS) and analyzed using 10x sequencing. Figure 5b illustrates Uniform manifold approximation and projection (UMAP) embedding of all cells colored by cell type suggesting equal overlap of stimulated and control cells. Figures 5c and 5d are graphs illustrating the Number of differentially expressed genes (log₂ fold change > 0.5 and p value < 0.05) for all cell types, which shows that the monocyte and macrophage subset have the highest number of differentially expressed genes in stimulated and untreated wounds. Figure 5e are three images showing UMAP embedding split by macrophages and monocytes (left) verified with the CytoTrace platform (middle), which identifies differentiated cell states within the monocyte cluster. RNA velocity (right), shown as the main gene-averaged flow, visualized by velocity streamlines projected onto the UMAP embedding of the monocyte cluster categorized by treatment group and labeled with three trajectories identified by the program (trajectory 1 (right) and 2 (middle), whereas trajectory 3 (left)). These data show that monocytes are less differentiated than macrophages, as expected. RNA velocity using Monocle 3 suggests three potential fates for macrophages and monocytes, starting with the initial node, marked with a circle in the right panel. Three distinct trajectories were observed, with stimulated cells clustering along trajectory 1 and 2, while trajectory 3 was mainly composed of unstimulated cells.

Figure 5f is an image showing UMAP embedding of macrophage and monocyte Seurat clusters, grouped by cells of similar differential expression, with a pro-regenerative cluster 2, outlined with a dotted black circle, shows that there are five transcriptionally distinct clusters. Cluster 0 consists mainly of macrophages and unstimulated cells. Clusters 1, 2 and 3 consist of stimulated monocytes and macrophages. Figure 3g is a Heatmap of the top differentially expressed genes in each Seurat cluster in Figure 3f showing that cluster 0 has a higher expression of genes associated with wound healing, whereas cluster 1, 2, and 3 have a higher expression of genes involved in the wound repair process. Figure 5h are Feature plots, split by treatment (stimulated) and control, of differentially expressed genes upregulated in cluster 2 in the macrophages and monocytes indicate that there is an enrichment for pro-regenerative markers localized around cluster 2 and trajectory 2, consisting primarily of stimulated macrophages.

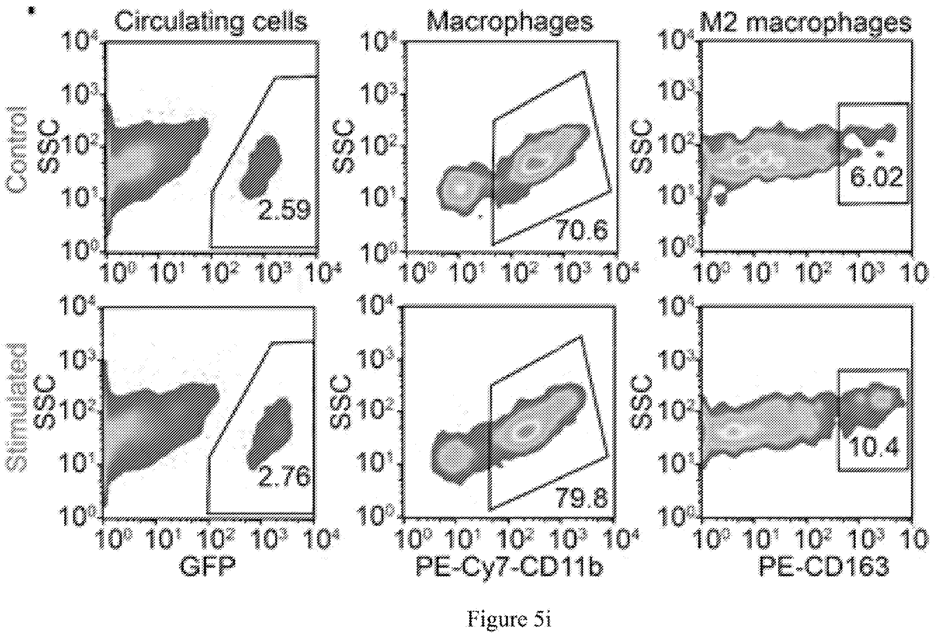

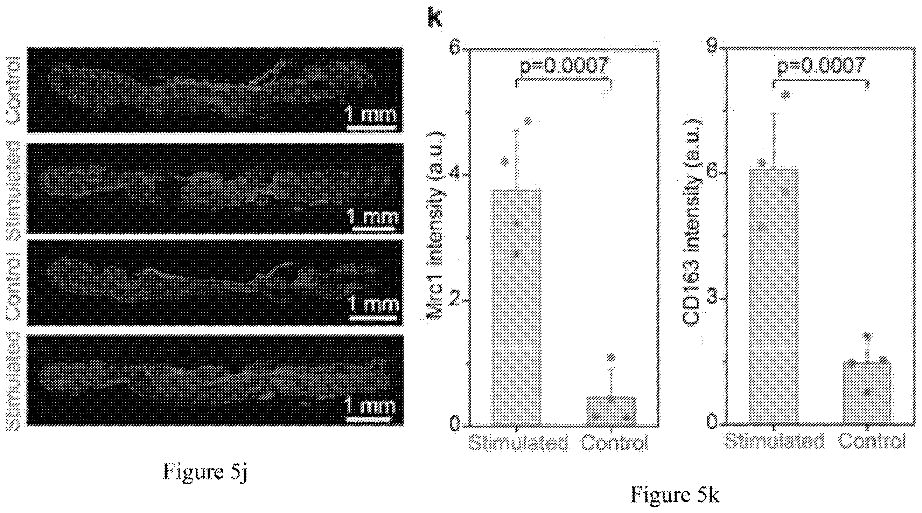

Figure 5i provides FACS plots for treatment and control groups of GFP-positive cells circulating in the parabiosis wound model verify a higher percentage of M2 macrophages in the stimulated group. Figures 5j and 5k provide a quantitative comparison of Mrc1 and CD163, respectively, from tissue with and without stimulation verifying M2 macrophage markers.

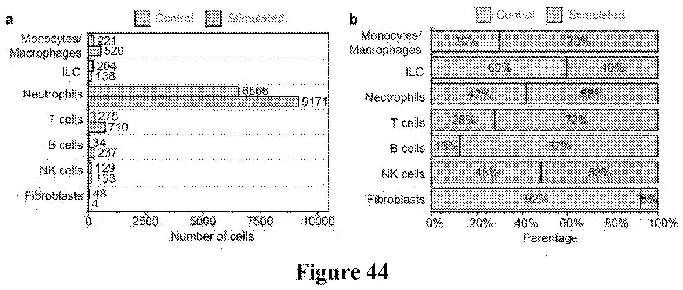

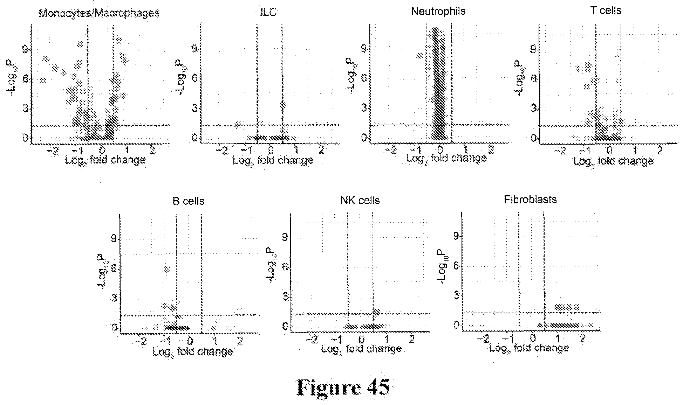

As shown in the figures above, in the present embodiments, due to the lightweight and untethered features of the smart bandage, it is possible to evaluate the long-term effects of electrical stimulation on circulating cells involved in wound repair using a complex parabiosis model (Duyverman, A. M., Kohno, M., Duda, D. G., Jain, R. K. & Fukumura, D. A transient parabiosis skin transplantation model in mice. Nat Protoc 7, 763-770, doi:10.1038/nprot.2012.032 (2012)). This would not have been possible previously, as with a conventional wired modality parabiosis under a long anesthesia regimen would not survive. To execute, performed was a parabiosis of five green fluorescence protein (GFP) positive mice to wild type (WT) mice. WT mice were wounded and either subjected to electrical stimulation or left untreated. Wound tissues from both groups were explanted on day 5 and their transcriptional profiles were analyzed by single-cell RNA sequencing (scRNA-seq) using the 10x Genomics Chromium platform as shown in Figures 5a. Of all the circulating inflammatory cells that were activated by the present smart bandage as shown in Figures 5b and Figure 44, monocytes and macrophages had the highest number of differentially expressed genes in electrically stimulated and untreated wounds as shown in Figures 5c, 5d and Figure 45. Even with many neutrophils present, the magnitude of differentially expressed genes did not reach statistical significance (Figures 44, 45). Similarly, while there were a higher number of B and T cells in the stimulated group, signifying greater recruitment of these cells from the circulation (Sirbulescu, R. F. et al. Mature B cells accelerate wound healing after acute and chronic diabetic skin lesions. Wound Repair Regen 25, 774-791, doi:10.1111/wrr.12584 (2017); Hofmann, U. et al. Activation of CD4+ T lymphocytes improves wound healing and survival after experimental myocardial infarction in mice. Circulation 125, 1652-1663, doi:10.1161/CIRCULATIONAHA.111.044164 (2012)), the overall number of cells was low and the amount of differentially expressed genes was nominal (Figures 44 and 45).

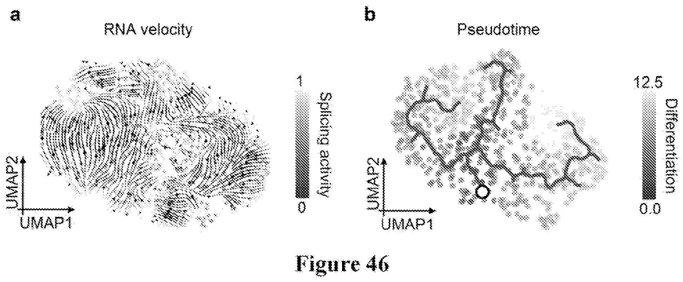

To specifically investigate the macrophages and monocytes, performed were a series of evaluations to validate and define the high number of differentially expressed genes observed. First, the macrophages and monocytes were re-embedded and CytoTRACE was used to confirm that the defined monocytes possessed less differentiated cell states based on the distribution of unique mRNA transcripts as shown in Figure 5e. Then overlaid were the stimulated and unstimulated macrophages and monocytes and performed RNA velocity and pseudotime analyses using scVelo and Monocle 3, respectively, to combine RNA velocity information with trajectory inference to compute a map of potential fates that the macrophages and monocytes can undertake in response to electrical stimulation. First used was scVelo to infer the root node and transcriptional directionality across the manifold based on mRNA splicing of the macrophages and monocytes. Found were three general transcriptional vector paths in which mRNA splicing could occur within individual cells, with a relatively higher amount of differentiated individual cells found on the left of the embedding and less differentiated cells found on the right (Figure 46a), further confirming CytoTRACE. Then performed was a pseudotime analysis using Monocle 3, using a root node identified with scVelo (marked with a circle in the right panel of Figure 5e) to infer terminal cell states (Bergen, V., Lange, M., Peidli, S., Wolf, F. A. & Theis, F. J. Generalizing RNA velocity to transient cell states through dynamical modeling. Nat Biotechnol 38, 1408-1414, doi:10.1038/s41587-020-0591-3 (2020)). This analysis once again revealed 3 distinct transcriptional trajectories, with stimulated cells clustered mainly along trajectory 1 (right) and 2 (middle), while trajectory 3 (left) was mainly composed of unstimulated cells as shown in Figures 5e and Figure 46b.

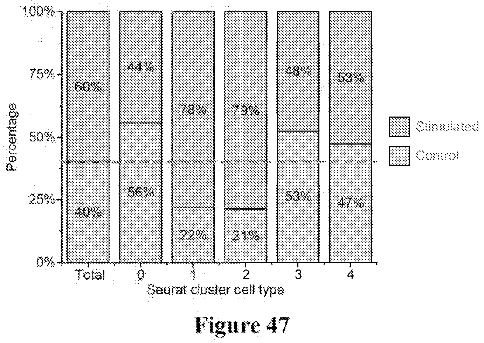

To further understand why the macrophages and monocytes had a higher amount of differentially expressed genes activated by the present smart bandage, performed was a uniform manifold approximation and projection (UMAP) based clustering which revealed 5 transcriptionally distinct clusters as shown in Figures 5f and Figure 47. Of the five clusters, cluster 0, consisting of both macrophages and unstimulated control cells, had a higher expression of genes such as Jun and Fn1 (Wernig, G. et al. Unifying mechanism for different fibrotic diseases. Proc Natl Acad Sci U S A 114, 4757-4762, doi:10.1073/pnas.1621375114 (2017); Wang, J. et al. High expression of Fibronectin 1 suppresses apoptosis through the NF-κB pathway and is associated with migration in nasopharyngeal carcinoma. Am J Transl Res 9, 4502-4511 (2017)), which have previously been associated with wound healing, whereas clusters 1, 2, and 3, consisting predominantly of stimulated monocytes and macrophages, demonstrated elevated expression of genes that are involved in the wound repair process, such as Cd74, Selenop, Apoe, Mrc1, Cd163, and Fabp5 (Farr, L., Ghosh, S. & Moonah, S. Role of MIF Cytokine/CD74 Receptor Pathway in Protecting Against Injury and Promoting Repair. Front Immunol 11, 1273, doi:10.3389/fimmu.2020.01273 (2020); Carlson, B. A. et al. Selenoproteins regulate macrophage invasiveness and extracellular matrix-related gene expression. BMC Immunol 10, 57, doi:10.1186/1471-2172-10-57 (2009); Lin, J. D. et al. Single-cell analysis of fate-mapped macrophages reveals heterogeneity, including stem-like properties, during atherosclerosis progression and regression. JCI Insight 4, doi:10.1172/jci.insight.124574 (2019); Huang, Z. H., Reardon, C. A. & Mazzone, T. Endogenous ApoE expression modulates adipocyte triglyceride content and turnover. Diabetes 55, 3394-3402, doi:10.2337/db06-0354 (2006)) (Fig. 5g).

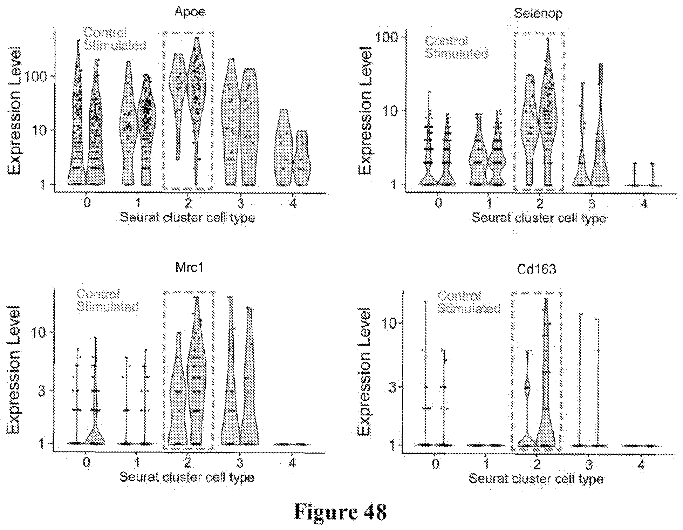

Interestingly, when the stimulated and control feature plots of highly expressed genes in macrophages and monocytes were analyzed, it was seen that cells with a strong enrichment for pro-regenerative markers, notably Cd163 and Mrc1 (CD206), as well as Selenop and Apoe, all localized around Seurat cluster 2 and trajectory 2 (middle), which primarily contained stimulated macrophages as shown in Figure 5h and Figure 48. Cd163 and Mrc1 (CD206) have been previously described as M2 anti-inflammatory macrophage markers (Martinez, F. O. & Gordon, S. The M1 and M2 paradigm of macrophage activation: time for reassessment. F1000Prime Rep 6, 13, doi:10.12703/P6-13 (2014)), and Selenop has been found to be anti-inflammatory, regulating macrophage invasiveness and other inflammatory mediators responsible for pathogen clearance and tissue repair, and is linked to M2-marcophage markers such as Stab1, Sepp1 and Arg163. Apoe has been also shown to enhance in vitro phagocytosis of macrophages, increasing muscle and soft tissue regeneration (Arnold, L. et al. CX3CR1 deficiency promotes muscle repair and regeneration by enhancing macrophage ApoE production. Nat Commun. 6, 8972, doi:10.1038/ncomms9972 (2015); Wang, Y. et al. Tissue-resident macrophages promote extracellular matrix homeostasis in the mammary gland stroma of nulliparous mice. Elife 9, doi:10.7554/eLife.57438 (2020)).

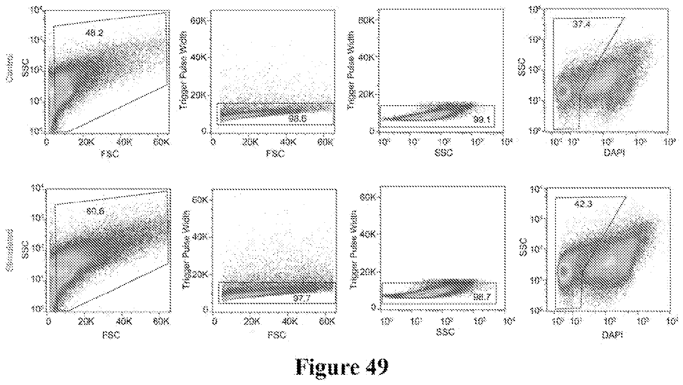

These transcriptional changes were further confirmed on the protein level, performing flow cytometry on GFP-positive cells circulating to wounds in our parabiosis model. Identified was a higher percentage of CD163-positive cells in stimulated wounds as compared to controls as shown in Figures 5i and Figure 49. This was further confirmed by immunofluorescent staining of healed tissue, with significantly higher CD163 and CD206 expression observed in stimulated wounds as compared to untreated wounds as shown in Figures 5j-5k).

These data suggest that electrical stimulation may drive macrophages towards a more regenerative phenotype and could underlie the accelerated wound healing observed in these pre-clinical studies. The high predominance of regenerative macrophages could be in part due to macrophages responding to the local micro-environmental stimuli. Modulating the cell membrane electric potential with electrical stimuli could activate more KATP ion channels, which has previously been shown to affect macrophage differentiation plasticity and function (Li, C., Levin, M. & Kaplan, D. L. Bioelectric modulation of macrophage polarization. Sci Rep 6, 21044, doi:10.1038/srep21044 (2016); Hoare, J. I., Rajnicek, A. M., McCaig, C. D., Barker, R. N. & Wilson, H. M. Electric fields are novel determinants of human macrophage functions. J Leukoc Biol 99, 1141-1151, doi:10.1189/jlb.3A0815-390R (2016)). Taken together, these pre-clinical studies attribute one mechanism by which electrical stimulation may coordinate and regulate macrophage functions, including those essential for microbial clearance and wound healing. The present smart bandage, in turn, can enable further biological discovery and allow for researchers to explore hypotheses that have been previously less studied, due to current treatment modality limitations and animal model complexities.

### Conclusions

In summary, the present embodiments provide a miniaturized smart bandage with dual channel continuous sensing of wound impedance and temperature, as well as a parallel stimulation circuit to deliver programmed electrical cues for accelerated wound healing. Through the integration of sensors and stimulators into one wearable patch as well as rational design of tissue interfacing hydrogel electrodes, the present wireless smart bandage enables: (i) active monitoring and closed-loop treatment of a wound, and (ii) accelerated healing through a pro-regenerative mode of action, activated by increased cellular proliferation and recruitment of cells involved in wound repair. This dual-modal integration advances the field of wound healing pathobiology, enabling for optimization of treatment modalities that would allow for better patient mobility and improvement in standard of care.

However, additional challenges exist, including production scalability (e.g. reduced cost, long-term storage), incorporation of additional sensors (e.g., metabolites, biomarkers, pH), and clinical translatability (e.g., biocompatibility, biofouling issues). While pre-clinical demonstrations showed proof of concept, future work involves extending the present smart bandage to a human-sized form factor and running preliminary tests in large animal models followed by human trials. In addition, it is possible to reduce the manufacturing cost of the clinical device to enable broad adoption within a payer system. Finally, the present device platform may also be adapted to other disease management, such as treatment of skin cancer and post-surgical scarring, enabling the next generation of closed-loop bioelectronic medicine.

### Example Methods

### - Fabrication of an example flexible circuit

Flexible printed circuit boards (FPCB) with custom designs are manufactured by commercial vendors with ISO 9001 certificate, e.g, PCBWay. The bill of materials includes passive components (capacitors, resistors, and diodes), a thermistor (103KT1608T-1P, Semitec), a near-field communication (NFC) ISO15693 sensor transponder with a programmable low-power microcontroller (MCU, RF430FRL152H, Texas Instruments), a crystal oscillator (SG-3040LC 32.7680KB3: PURE SN, Epson Timing), and two operational amplifiers (TSV620AILT, STMicroelectronics). All circuit components were soldered using tin-lead solder paste (Sn63/Pb37, melting point: 183 oC, 247 Solder) by hot-air blowing.

To avoid chronic electrochemical corrosion of the electroless nickel immersion gold (ENIG) electrodes on the backside of the smart bandage board (two for stimulation, two for sensing), silver/silver chloride (Ag/AgCl) conducting paste (CI-4040, Nagase ChemteX) was printed onto each electrodes using blades and cured on hot plate at 100 oC for 10 min. The electrode area was further encapsulated by an elastomeric polyurethane coating (Clear Flex 50, Mix Ratio: 1A/2B wt/wt, Smooth-On) and cured at 70 oC for 15 min. The hydrogel interface electrodes were then placed onto corresponding Ag/AgCl electrodes for sensing and stimulation purposes.

For one example smart bandage preparation, the PCB board manufacturing and shipping takes about 7 days, and the soldering and gel preparation steps take another day. The total production time is about 8 days. The electronics themselves are also completely reusable. For a batch of 50 FPCB boards, the total expense is typically ~$150. Each device would cost ~$3. All the electronic components being soldered onto the device cost ~$13. For the hydrogel electrode, the material cost is ~$2 per device. The overall cost is ~$18 per smart bandage.

The resonant frequency and quality factor of the antenna was measured using a portable vector network analyzer (VNA, miniVNA Tiny Plus 2). The resonant frequency was determined by the peak location of the impedance amplitude. The quality factor was calculated by resonant frequency divided by the 3 dB bandwidth.

### - Synthesis of an example conducting adhesive hydrogel

In a typical synthesis, 40 mg of poly(3,4-ethylenedioxythiophene) polystyrene sulfonate dry pellet (Orgacon^{™} DRY5, Agfa), 300 mg of N-isopropylacrylamide (NIPAM, Sigma-Aldrich), 24 mg of acrylamide (AAm, Sigma-Aldrich), and 1 mg of N,N'-methylenebisacrylamide (MBAA, Sigma-Aldrich) were mixed and dissolved in 2 mL of water by grinding in an agate mortar to form a viscous suspension. Prior to the gel initiation, 50 µL of 30% (v/v) hydrogen peroxide (H₂O₂, Fisher Scientific) and 50 µL of 20% (wt/v) ascorbic acid (AA, Sigma-Aldrich) were added sequentially to the monomer mixture and quickly mixed before pouring into Teflon molds for subsequent gelation at room temperature.

For the control sample without PEDOT:PSS, the same procedure was performed except for the absence of PEDOT:PSS pellet in the initial mixture. For the study of AAm content on the overall lower critical solution temperature (LCST) behavior, the initial amount of AAm was varied from 0 mg, 18 mg, 24 mg, and 30 mg. For the study of electrochemical properties, the initial amount of PEDOT:PSS was varied from 0 mg, 10 mg, 20 mg, 30 mg, and 40 mg. For the study of crosslinking density, the initial amount of MBAA was varied from 0.3 mg, 1 mg, 3 mg, and 10 mg.

The hydrogels were stored in sealed and moisturized bags at 4 °C to avoid evaporation of water. Under this storage condition, the gels can maintain a stable hydration level for at least 4 weeks. Longer term storage was not evaluated. For animal experiments, hydrogels were cut into blocks with lateral dimensions of 8 mm by 4 mm to ensure consistent contact areas with the skin. The hydrogel electrodes were placed at the periphery of the wound with one pair for stimulation and another pair for impedance sensing. Because of the position of the hydrogel (periphery of the wound), the hydrogel was not in direct contact with the wound fluid itself. As a result, severe biofouling was not encountered in example experiments. The skin hydration level of the mice is typically stable in controlled housing and laboratory environments. Regarding the contact pressure, because of the high adhesion of our hydrogel electrode, it can be attached stably to the skin with consistent contact impedance. Therefore, there was no need to control for contact pressure in these examples.

### - Example electrochemical characterizations

Electrochemical impedance spectroscopy (EIS) was performed by using the hydrogel as the working electrode, platinum as the counter electrode, silver/silver chloride (Ag/AgCl) as the reference electrode, and PBS as the electrolyte. Impedance and phase angle as functions of frequency were acquired by a Bio-Logic VSP-300 workstation with a sine wave signal amplitude of 10 mV. Chronoamperometry was performed on the same potentiostat by delivering biphasic square wave pulses with 50 ms for each phase and 100 mV amplitudes with currents being recorded simultaneously.

Conductivity measurements were carried out using a four-point probe method using a Keithley 4200 SC semiconductor analyzer. For the measurement of impedance change over strain, the conducting hydrogel was mounted onto a home-made automated stretcher when the impedance values at different strain levels would be recorded using a LCR meter (Keysight Technologies, E4098A).

### - Example mechanical characterizations

Uni-directional tensile tests of the hydrogel electrodes were performed on an Instron 5565 at a strain rate of 10 mm min⁻¹. To measure the interfacial energy, hydrogel samples and surfaces for adhesion (e.g., metal, plastic, rubber, and skin) were glued using cyanoacrylate (Krazy Glue) onto Kapton polyimide films as a stiff backing. After adhering the hydrogel onto the surface of interest, the adhesion was tested by the standard 180-degree peel test with the Instron machine. All tests were conducted with a constant peeling speed of 10 mm min⁻¹. Interfacial toughness was calculated by dividing two times the plateau by the width of the tissue sample. Differential scanning calorimetry (DSC) was conducted using a TA Instruments Q2000 DSC. Rheological measurements were performed using a TA Instruments ARES-G2 rheometer.

### - Example hydrogel biocompatibility assay

All tissue sample histology slides were examined via light microscopy by the study pathologist. Wound sites were semi-quantitatively scored per the criteria of Figure 24.

Semi-quantitative scores were assigned based on the representative site response observed over six noncontiguous representative high-power microscope fields at the host/test sample interface. The Test Sample (with gel) and Control Sample (without gel) scored results are documented by animal, as seen in Fig. 25. Sample Reactivity Score calculations are documented and categorized. Representative low and high magnification implant site photomicrographs are contained in Fig. 25. After scoring of each implant site sample or subsample slide section, the following steps were performed. Polymorphonuclear cells, lymphocytes, plasma cells, macrophages, giant cells, and necrosis scores assigned for each implant were totaled and multiplied by 2 (Subtotal A). Neovascularization, fibrosis, and fatty infiltration scores assigned for each implant were totaled (Subtotal B). Subtotal A and Subtotal B were added together for a total implant score (Total). For each Test or Control Sample type, the Total values were added together (Group Total). Each Group Total was averaged (Group Average) by the appropriate number of scored implants. The Control Group Average was subtracted from the Test Group Average to derive the final average test result (Test Sample Relative Score). A resulting negative difference was recorded as zero, per protocol. The Test Sample Relative Score, per part c in Fig. 25, was used to categorize the Test Sample.

The herein described subject matter sometimes illustrates different components contained within, or connected with, different other components. It is to be understood that such depicted architectures are illustrative, and that in fact many other architectures can be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" each other such that the desired functionality is achieved, irrespective of architectures or intermedial components. Likewise, any two components so associated can also be viewed as being "operably connected," or "operably coupled," to each other to achieve the desired functionality, and any two components capable of being so associated can also be viewed as being "operably coupleable," to each other to achieve the desired functionality. Specific examples of operably coupleable include but are not limited to physically mateable and/or physically interacting components and/or wirelessly interactable and/or wirelessly interacting components and/or logically interacting and/or logically interactable components.

With respect to the use of plural and/or singular terms herein, those having skill in the art can translate from the plural to the singular and/or from the singular to the plural as is appropriate to the context and/or application. The various singular/plural permutations may be expressly set forth herein for sake of clarity.

It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.).

Likewise, software implementations of the described methods could be accomplished with standard programming techniques with rule-based logic and other logic to accomplish the various connection steps, processing steps, comparison steps, and decision steps.

It will be further understood by those within the art that if a specific number of an introduced claim recitation is intended, such an intent will be explicitly recited in the claim, and in the absence of such recitation, no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to inventions containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations).

Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general, such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

Further, unless otherwise noted, the use of the words "approximate," "about," "around," "substantially," etc., mean plus or minus ten percent.

## Claims

1. An apparatus comprising:
a smart bandage (102) that includes:
reversible skin electrodes comprising a polymer-based dual-conducting hydrogel (106) configured to provide reversible skin adhesion for the smart bandage; and
a flexible electronics (104) having processing circuitry and a wireless interface for communicating with external processors.

2. The apparatus of claim 1, wherein the processing circuitry includes a built-in closed-loop feedback to receive information from sensors and to actively deliver precise electrical stimulation.

3. The apparatus of claim 2, wherein the processing circuitry is configured to deliver stimulation in response to the detection of early infection after hemostasis has been achieved.

4. The apparatus of claim 1, wherein the electrodes possess robustness, and low impedance for electrical stimulation.

5. The apparatus of claims 1-4, wherein the polymer-based dual-conducting hydrogel comprises an electronic-ionic dual conducting polymer complex.

6. The apparatus of claim 5, wherein the polymer complex comprises polymerized biocompatible (meth)acrylate monomers in the presence of conducting polymers based on PEDOT:PSS.

7. The apparatus of claims 1-6, wherein the polymer-based dual-conducting hydrogel incorporates adhesive interfacial chemistry comprising hydrogen bonding or metal-coordination, and tuned nanoscopic intermolecular interaction.

8. The apparatus of claims 1-7, wherein the flexible electronics comprises biosensor technology for impedance and temperature.

9. The apparatus of claims 1-8, wherein the flexible electronics comprises electrical stimulation controlled by the processing circuitry.

10. The apparatus of claims 1-9, wherein the flexible electronics includes an antenna for wireless energy harvesting or a rechargeable battery for energy supply.

11. The apparatus of claims 1-10, wherein the flexible electronics comprises a ring oscillator for AC impedance measurement.

12. The apparatus of claims 1-11, wherein the flexible electronics comprises a temperature sensitive resistor for temperature sensing.

13. The apparatus of claims 1-12, wherein the wireless interface comprises a Bluetooth unit.

14. The apparatus of claims 1-13, wherein the polymer-based dual-conducting hydrogel is coupled to the processing circuitry through gold wires.

## Patentansprüche

1. Einrichtung, umfassend:
eine intelligente Bandage (102), die Folgendes einschließt:
umkehrbare Hautelektroden, die ein dual leitfähiges Hydrogel (106) auf Polymerbasis umfassen, das konfiguriert ist, um eine umkehrbare Hauthaftung für die intelligente Bandage bereitzustellen; und
eine flexible Elektronik (104), die eine Verarbeitungsschaltlogik und eine drahtlose Schnittstelle zur Kommunikation mit externen Prozessoren aufweist.

2. Einrichtung nach Anspruch 1, wobei die Verarbeitungsschaltlogik eine eingebaute geschlossene Rückkopplungsschleife einschließt, um Informationen von Sensoren zu empfangen und aktiv eine präzise elektrische Stimulation abzugeben.

3. Einrichtung nach Anspruch 2, wobei die Verarbeitungsschaltlogik konfiguriert ist, um eine Stimulation als Reaktion auf die Erkennung einer frühen Infektion abzugeben, nachdem eine Hämostase erreicht wurde.

4. Einrichtung nach Anspruch 1, wobei die Elektroden robust sind und eine niedrige Impedanz für die elektrische Stimulation aufweisen.

5. Einrichtung nach Anspruch 1 bis 4, wobei das dual leitfähige Hydrogel auf Polymerbasis einen elektronisch-ionischen dual leitfähigen Polymerkomplex umfasst.

6. Einrichtung nach Anspruch 5, wobei der Polymerkomplex polymerisierte biokompatible (Meth)acrylatmonomere in Gegenwart von leitfähigen Polymeren basierend auf PEDOT:PSS umfasst.

7. Einrichtung nach Anspruch 1 bis 6, wobei das dualleitende Hydrogel auf Polymerbasis eine Klebstoffgrenzflächenchemie umfasst, die eine Wasserstoffbindung oder Metallkoordination und eine abgestimmte nanoskopische intermolekulare Wechselwirkung umfasst.

8. Einrichtung nach Anspruch 1 bis 7, wobei die flexible Elektronik eine Biosensorik für Impedanz und Temperatur umfasst.

9. Einrichtung nach Anspruch 1 bis 8, wobei die flexible Elektronik eine elektrische Stimulation umfasst, die von der Verarbeitungsschaltlogik gesteuert wird.

10. Einrichtung nach Anspruch 1 bis 9, wobei die flexible Elektronik eine Antenne zur drahtlosen Energiegewinnung oder eine wiederaufladbare Batterie zur Energieversorgung einschließt.

11. Einrichtung nach Anspruch 1 bis 10, wobei die flexible Elektronik einen Ringoszillator zur AC-Impedanzmessung umfasst.

12. Einrichtung nach Anspruch 1 bis 11, wobei die flexible Elektronik einen temperaturempfindlichen Widerstand zur Temperaturerfassung umfasst.

13. Einrichtung nach Anspruch 1 bis 12, wobei die drahtlose Schnittstelle eine Bluetooth-Einheit umfasst.

14. Einrichtung nach Anspruch 1 bis 13, wobei das dual leitfähige Hydrogel auf Polymerbasis über Golddrähte mit der Verarbeitungsschaltlogik gekoppelt ist.

## Revendications

1. Appareil comprenant :
un bandage intelligent (102) qui comporte :
des électrodes cutanées réversibles comprenant un hydrogel à double conductivité à base de polymère (106) conçu pour fournir une adhérence cutanée réversible pour le bandage intelligent ; et
un équipement électronique flexible (104) ayant un ensemble de circuits de traitement et une interface sans fil pour communiquer avec des processeurs externes.

2. Appareil selon la revendication 1, dans lequel l'ensemble de circuits de traitement comporte une rétroaction à boucle fermée intégrée pour recevoir des informations provenant de capteurs et pour administrer activement une stimulation électrique précise.

3. Appareil selon la revendication 2, dans lequel l'ensemble de circuits de traitement est configuré pour administrer une stimulation en réponse à la détection d'une infection précoce après l'obtention d'une hémostase.

4. Appareil selon la revendication 1, dans lequel les électrodes possèdent une robustesse et une faible impédance pour la stimulation électrique.

5. Appareil selon les revendications 1 à 4, dans lequel l'hydrogel à double conduction à base de polymère comprend un complexe polymère à double conduction électronique-ionique.

6. Appareil selon la revendication 5, dans lequel le complexe polymère comprend des monomères (méth)acrylate biocompatibles polymérisés en présence de polymères conducteurs à base de PEDOT:PSS.

7. Appareil selon les revendications 1 à 6, dans lequel l'hydrogel à double conduction à base de polymère incorpore une chimie interfaciale adhésive comprenant une liaison hydrogène ou une coordination métallique, et une interaction intermoléculaire nanoscopique ajustée.

8. Appareil selon les revendications 1 à 7, dans lequel l'équipement électronique flexible comprend une technologie de biocapteurs pour l'impédance et la température.

9. Appareil selon les revendications 1 à 8, dans lequel l'équipement électronique flexible comprend une stimulation électrique commandée par l'ensemble de circuits de traitement.

10. Appareil selon les revendications 1 à 9, dans lequel l'équipement électronique flexible comporte une antenne pour la récupération d'énergie sans fil ou une batterie rechargeable pour l'alimentation en énergie.

11. Appareil selon les revendications 1 à 10, dans lequel l'équipement électronique flexible comprend un oscillateur en anneau pour la mesure d'impédance CA.

12. Appareil selon les revendications 1 à 11, dans lequel l'équipement électronique flexible comprend une résistance sensible à la température pour la détection de la température.

13. Appareil selon les revendications 1 à 12, dans lequel l'interface sans fil comprend une unité Bluetooth.

14. Appareil selon les revendications 1 à 13, dans lequel l'hydrogel à double conduction à base de polymère est couplé à l'ensemble de circuits de traitement par des fils d'or.
